# EUROPEAN PATENT APPLICATION

(11) **EP 3 882 632 A1**
(43) Date of publication of application: **22.09.2021**
(21) Application number: 20163892.1
(22) Date of filing: 18.03.2020
(51) Int. Cl.: G01N 33/542, G01N 33/68

(54) **NOVEL ION CONDUCTING CHANNEL FUSION SUBUNITS AND METHODS OF USE THEREOF**

(71) Applicant: UNIVERSITE DE BORDEAUX, 33000 Bordeaux (FR); UNIVERSITE DE MONTPELLIER, 34090 Montpellier (FR); Institut Polytechnique de Bordeaux, 33402 Talence Cedex (FR); CENTRE NATIONAL DE LA RECHERCHE SCIENTIFIQUE, 75794 Paris Cedex 16 (FR); INSTITUT NATIONAL DE LA SANTE ET DE LA RECHERCHE MEDICALE - INSERM, 75013 Paris (FR)
(72) Inventor: PERCHERANCIER, Yann, 33140 Villenave d'Ornon (FR); COMPAN, Vincent, 34980 Combaillaux (FR); RASSENDREN, Francois, 34070 Montpellier (FR); OLLIVIER, Matthias, 69330 Myzieu (FR); CHAPPE, Yann, 33650 Saint Selve (FR)
(74) Representative: Lecca, Patricia S.

(57) **Abstract**

The present invention is directed to a channel fusion subunit comprising an ion conducting channel subunit bound to a probe, wherein the probe comprises an ion sensor bound in between to at least one bioluminescent donor molecule and at least one fluorescent acceptor molecule, wherein the probe is bound to the N-terminal, C-terminal, or within an intracellular or extracellular loop of said channel subunit via either the bioluminescent donor molecule or the fluorescent acceptor molecule, and wherein said bioluminescent donor molecule and fluorescent acceptor molecule are selected so that the emission spectrum of the bioluminescent donor molecule overlaps with the absorbance spectrum of the acceptor molecule, making it possible the nonradiative energy transfer between the bioluminescent energy donor and the fluorescent acceptor through nonradiative dipole-dipole coupling.

## Description

### FIELD OF THE INVENTION

The present invention relates generally to novel ion conducting channel fusion subunits and/or novel ion conducting channel fusions, nucleic acids encoding such channel fusions and channel subunit fusions, amino acid sequences, recombinant cells and expression vectors comprising such channel fusions and channel subunit fusions, as well as method of use thereof particularly for high throughput screening (HTS) methods for screening inhibitors or activators of the ion conducting channels.

### BACKGROUND OF THE INVENTION

Life of higher organisms including humans requires quick and efficient transmission of electric and chemical signals that synchronize myriad diverse cellular processes. The interior of animal and plant cells is electrically negative with respect to the exterior. The magnitude of this potential difference is generally between 5 and 90 mV, with most of the potential being developed across the cell membrane. The transmembrane potential of a given cell is set by the balance of the activities of ion transporters and channels which create and maintain the electrochemical gradient, and the activities of ion channels, passive diffusion and other factors, that allow ions to flow across the plasma membrane. Electrical signals are vital for cellular functions and are mediated primarily by ion channels or ion conducting channels, a specialized group of proteins which span the membranes of all living cells and which are permeable to electrically charged atoms (ions) present in the body. In a simple sense, ion conducting channels can be considered the biological equivalent of transistors as they respond to stimuli by switching between "on" (open) and "off" (closed) states, thereby controlling the passage of electric current across the cell membrane. In doing so, ion conducting channels control the electrical state of cells and allow such fundamental processes as the beating of the heart, contraction of muscles and information processing in the nervous system. Since ion conducting channels allow the movement of ions across cell membranes, they are completely responsible for the entire range of electrical signaling within the living system. In addition, ion channels either directly, by allowing calcium flux, or indirectly, by modulating membrane potential, affect the intracellular calcium concentration; this in turn regulates a variety of functions, including energy production, synaptic transmission, muscle contraction, hormone secretion and gene transcription.

Specifically, ion conducting channels can be defined as pore-forming proteins that allow certain specific types of ions to pass through the channel by their electrochemical gradient to control slight voltage difference between plasma membranes of cells. These ion conducting channels are present in cell membrane of all living cells. Ion conducting channels can be divided into chloride ion channel family, potassium ion channel family, sodium ion channel family, calcium ion channel family, proton channel family and universal ion channel family in accordance with types of ions.

Given the central and pivotal role of ion conducting channels in many physiological processes, they represent a target class with enormous potential for intervention in a wide range of disease states. Indeed, the successful introduction of numerous ion conducting channel drug classes (*e.g*., calcium antagonists, sodium channel blockers, γ-aminobutyric acid or GABA receptor potentiators and sulphonylureas) onto the market has firmly established ion conducting channels as valid drug targets. However, most marketed ion conducting channel drugs that were discovered in the pre-molecular era, *i.e.,* first-generation ion channel drugs, are relatively nonselective, often exhibit dose-limiting side effects and are rarely 100% effective. Thus, there remains a clear need for improved ion conducting channel drugs, *i.e.,* new-generation ion channel drugs, that are more selective and that target ion conducting channels with fewer side effects and improved efficacy. Several diseases have been associated with ion conducting channel functionality. For instance, diseases resulting from ion conducting channel dysfunctions in the central nervous system include anxiety, depression, epilepsy, insomnia, memory problems and chronic pain. Other diseases resulting from ion conducting channel dysfunctions include cardiac arrhythmia, and type II diabetes.

The early methods developed to measure the activity of ion conducting channels were based on live cell recordings. Overexpression of the ion conducting channel of interest was often required for such approaches, which changes the cellular physiological state with unpredictable consequences on channel activity. This leads in many instances to toxicity for cell and their apoptosis. In these methods, the activity of the ion channels was measured mainly by patch-clamp or fluorescence.

The patch-clamp systems involve an electrophysiology-based measurement. An electrode is dipped into the cell and a reference electrode is outside in the solution containing the cell. When ions go through the channels, a current is generated across the membrane and is measured by the electrodes. If the channels are non-functional or blocked by compounds, no current will be recorded. The fluorescence-based systems use ion-specific (or pH-dependent) fluorescent molecules. However, such ion-specific fluorescent molecules do not exist yet for all ions. In order to overcome the drawbacks of the cell-based methods, cell-free methods have been developed still based on patch-clamp or fluorescence measures. In these cell-free methods, ion conducting channels are expressed, purified and reconstituted in model membranes, such as droplet interface bilayer (DIB), possibly in a microfluidic platform.

In order to discover new targets for therapy and new pharmaceutical compounds targeting ion channels, it is useful to be able to screen chemical libraries of numerous compounds by automated, high-throughput assays. However, the above cell-based or cell-free methods have limitations that do not facilitate a high-throughput screening (HTS). Indeed, most current HTS methods involve compounds under flow, typically in microfluidic or millifluidic devices. Thus, this precludes the use of patch-clamp methods due to the use of electrodes. A major limitation of the patch clamp technique is its low throughput. Typically, a single, highly trained operator can test fewer than ten compounds per day using the patch clamp technique. Furthermore, the technique is not easily amenable to automation, and produces complex results that require extensive analysis. The use of fluorescence-based methods is also not convenient since a fluorescent smear is observed under flow of a fluorescent compound which basically generates noise and limits the readout.

Fluorescence resonance energy transfer (FRET) and Bioluminescence resonance energy transfer (BRET) techniques have been developed to monitor protein-protein interactions, for drug discovery and HTS methods.

International publication No. WO2016/131832 describes first generation of intramolecular BRET probes comprising a transient receptor potential (TRP) channel subunit fused to a bioluminescent donor molecule and a fluorescent acceptor molecule either on the C-terminal or N-terminal extremity of the channel subunit. Such BRET probes allowed monitoring transient receptor potential (TRP) channel structural conformational changes and TRP channel activation in living cells. However, while these first generation of BRET probes are only efficient in measuring the change of conformation of ion channels, they do not always show in a reliable fashion actual activation or inhibition of the ion conducting channels since the change of conformation of the ion channels is not necessarily connected to actual activations or inhibitions.

One challenge to increase the discovery rate of drugs was thus to provide a method allowing measuring the passage of ions through membrane-imbedded channels in HTS methods. The present application is thus directed to novel BRET probes allowing to reliably measure activation or inhibition of ion conducting channels by measuring the actual passages of ions using BRET techniques and methods of use of these new probes for drug screening and mapping of signal transduction pathways.

### SUMMARY OF THE INVENTION

The present invention relates to nucleic acids encoding novel fusion proteins as well as the novel fusion proteins comprising ion conducting channel fusions or novel ion conducting channel fusion subunits. These fusions comprise an ion conducting channel, or a subunit thereof bound in C-terminal, N-terminal, or within an intracellular or extracellular loop, to a probe which comprising an ion sensor bound in between or sandwiched between at least one bioluminescent donor molecule and at least one fluorescent acceptor molecule, and wherein the ion sensor is configured to undergo a conformational change in the presence of an ion transported by the channel subunit.

The present invention also relates to expression vectors comprising nucleotide sequences encoding the novel ion conducting channel fusions or novel ion conducting channel fusion subunits, recombinant cells comprising such expression vectors, process for the production of ion channel subunit, as well as to the fusion proteins and fusion subunits obtainable by said process.

This invention further relates to a method for screening a candidate compound capable of modulating the ion flux through the ion conducting channel, and thus activating and/or inhibiting said channel by with measuring a variation of bioluminescent resonance energy transfer.

### BRIEF DESCRIPTION OF THE FIGURES

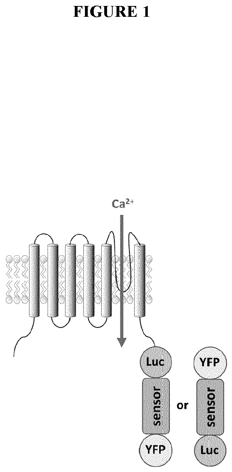

**Figure 1**: is a schematic representation of a novel intramolecular BRET probe using a putative 6-transmembrane domain Calcium channel as an example. The sensor sandwiched by Luc and YFP in both orientations is fused to the C-terminus of the channel. The fusion of the ion BRET biosensor to the N-terminus of the channel will also be constructed.

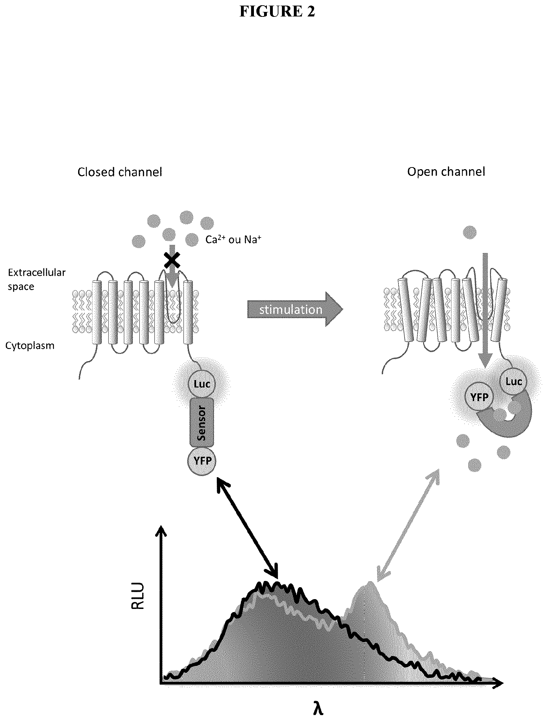

**Figure 2**: is a schematic representation of a novel BRET probe allowing to measure the TRP channel activity (6 transmembrane domains) by measuring the amount of ions diffusing under the channel during its opening via the BRET probe sensitive to ions which is fused in C-terminal to the channel of interest. During the passage of the ions across the pore of the opened channel, the ions diffuse towards the cytoplasm and are caught at the exit of the pore by the BRET probe which is fused to the channel of interest. The BRET probe will undergo a conformation change which will be reflected on the measure of the BRET signal. The groups Luc and YFP may be placed on either side of the ion sensor and/or may be fused in N-terminal of the channel of interest.

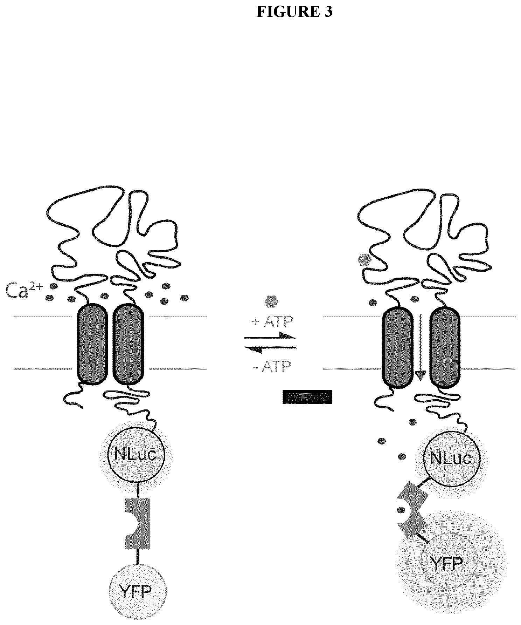

**Figure 3****:** is a schematic representation of a BRET probe of 2^{nd} generation allowing to measure the activity of P2X channel (2 transmembrane domains) via the detection of calcium ions passing through the channel during its opening and which are bound to the BRET probe fused to the C-terminal tail of the P2X receptor. During the passage of the calcium ions across the pore of the opened channel, the ions diffusing towards the cytoplasm are caught at the exit of the pore by the BRET probe which is fused to the channel of interest. This BRET probe will undergo a conformational change caused by the binding of the ions, which will be reflected by an increase of the BRET signal.

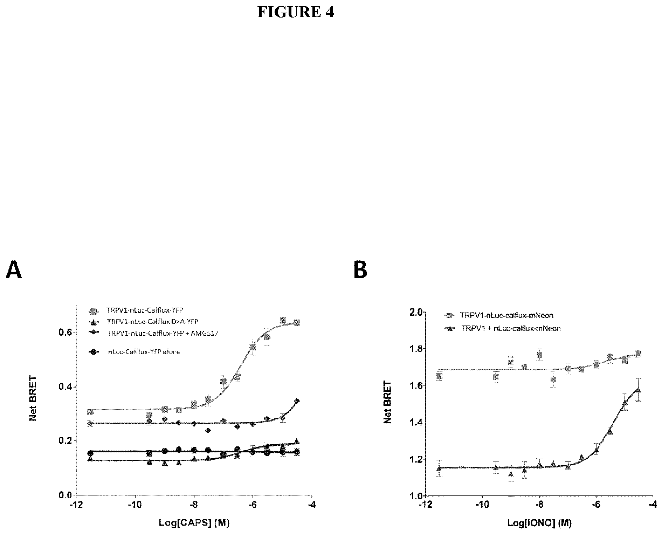

**Figure 4****:** Dose-response to Capsaïcine (CAPS) and lonomycine (IONO) as measured in HEK293T cells transitory expressing TRPV1-nLuc-calflux-YFP or TRPV1-nLuc-calflux-mNeonGreen probe, the TRPV1-nLuc-calflux D13A/D51A-YFP (TRPV1-nLuc-CalfluxD>A-YFP) probe or the nLuc-Calflux-mNeonGreen probe alone without (A) or with (B) TRPV1.

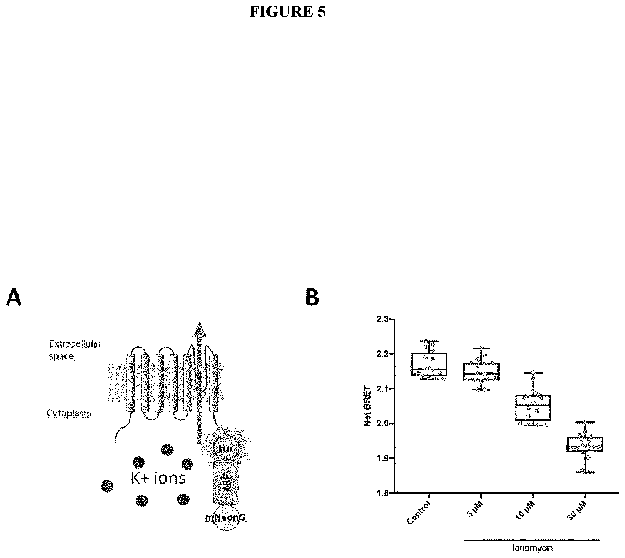

**Figure 5**: is a schematic representation of the SK3-nLuc-KBP-mNeonGreen probe (A) and the measure of BRET signal as obtained during the activation of the channel in presence of lonomycine (B).

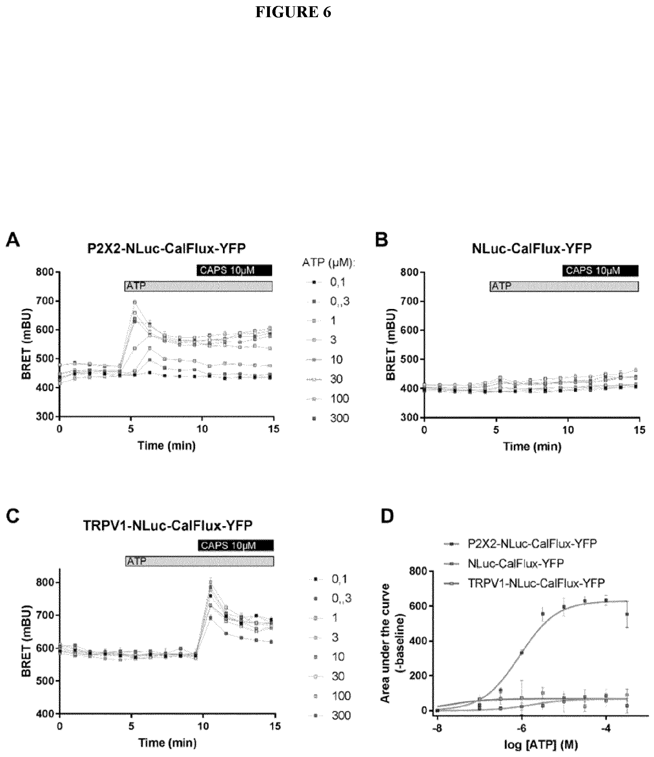

**Figure 6**: shows the dose-response of ATP as measured in HEK293T cells transiently expressing P2X2-nLuc-Calflux-YFP probe (A) or nLuc-Calflux-YFP probe (B) or TRPV1-nLuc-Calflux-YFP probe(C). Cells are stimulated with increasing doses of ATP 5 min after the addition of coelenterazine H. At the end of the reading, cells are stimulated with capsaïcine (CAPS), an agonist of TRPV1. These data allowed us to generate dose response and to determine the EC50 of the P2X2-nLuc-Calflux-YFP probe for ATP (D).

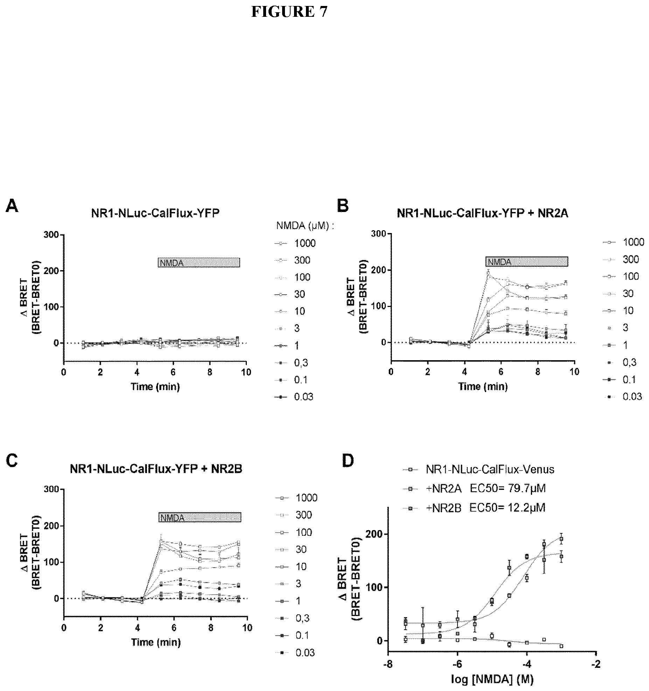

**Figure 7****:** Dose-response for NMDA as measured in HEK293T cells transiently expressing the NR1-nLuc-Calflux-YFP probe alone (A) or coexpressed with NR2A (B) or NR2B (C) subunits. Cells are stimulated with increasing doses of NMDA 4 min after the addition of coelenterazine H. With these data, we generated dose-response and determine the EC50 for NMDA of heteromeric receptors comprising NR2A or NR2B subunits (D).

### DETAILED DESCRIPTION

Recombinant protein, cell culture, and molecular cloning techniques utilized in the present invention are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T. A. Brown (editor), Essential Molecular Biology: A Practical Approach, Volumes 1 and 2, IRL Press (1991), D. M. Glover and B. D. Hames (editors), DNA Cloning: A Practical Approach, Volumes 1-4, IRL Press (1995 and 1996), and F. M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J. E. Coligan et al. (editors) Current Protocols in Immunology, John Wiley & Sons (including all updates until present).

The terms "bioluminescent donor molecule" refer to any molecule able to generate luminescence following either action on a suitable substrate, or its own excitation by an external source.

The terms "fluorescent acceptor molecule" refer to any compound which can accept energy emitted as a result of the activity of a bioluminescent donor molecule and re-emit it as light energy.

Bioluminescent resonance energy transfer (BRET) refers a proximity assay based on the non-radiative transfer of energy between the bioluminescent donor molecule and any suitable fluorescent acceptor molecule. Non-radiative transfer means transfer of energy from an energy donor to an energy acceptor without the emission of light by the donor.

The terms "spatial location" refer to the three-dimensional positioning of the bioluminescent donor molecule relative to the acceptor molecule which changes as a result of the compound binding to the voltage-dependent ion channel or of the change in specific physical parameters.

The terms "dipole orientation" refer to the direction in three-dimensional space of the dipole moment associated either with the donor molecule and/or the acceptor molecule relative to their orientation in three-dimensional space. The dipole moment is a consequence of a variation in electrical charge over a molecule. The resulting resonance energy transfer is an electromagnetic phenomenon in which quantum energy is transferred non-radiatively from an excited bioluminescent donor to a fluorescent acceptor molecule within close proximity.

The terms "resonance energy transfer" refer to the fact that energy transfer is by means of intermolecular dipole-dipole coupling, that is, the process does not involve emission and reabsorption of photons. The donor typically emits at shorter wavelengths that overlap with the absorption spectrum of the acceptor molecule.

The terms "protein-protein interaction" or "binding" refer to the specific complementary recognition and association of two proteins with a dissociation constant, Kd of preferably 10 ⁻⁵M, more preferably 10⁻⁷M, most preferably 10⁻⁹M or less.

Modulators, activators or inhibitors are any agents capable of altering the functional activity of ion channel within a cell and may represent a channel opener (functional agonist), a channel blocker (functional antagonist).

As used herein, the terms "fusion channel unit(s)" or "fusion channel(s)" generally refer to a polypeptide or protein channel or channel subunit that is comprised of two or more amino acid sequences, wherein each amino acid sequence encodes a protein or a portion thereof, wherein the two or more amino acid sequences are not found linked in nature, and wherein the two or more amino acid sequences are physically linked by a peptide bond.

As used herein, the term "domain" refers to a region of a protein that is at least 2 amino acids less than the whole protein and which retains at least the biological activity of the whole protein. A domain may range in size from 10-1000 amino acids in length, e.g. 50-60 amino acids, 100-400 amino acids or 200-300 amino acids. Domain refers to a functional unit of a complete protein having a biological activity of the complete protein. For example, the domain of a protein useful according to the invention may refer to a region of a protein that binds to a second protein.

The terms "more sensitive" refer to a greater change in resonance energy transfer ratio between the bioluminescent donor molecule and the fluorescent acceptor molecule.

The term "contacting" refers to the addition of a candidate molecule, a series of candidate molecules, and/or samples capable of activating or inhibiting the ion conducting channel and which may thus be screened by HTS methods using the new-generation BRET probe.

By "substantially purified" or "purified" is meant an ion conducting channel or channel subunit which has been separated from one or more lipids, nucleic acids, other polypeptides, or other contaminating molecules with which it is associated in its native state. It is preferred that the substantially purified polypeptide is at least 60% free, more preferably at least 75% free, and more preferably at least 90% free from other components with which it is naturally associated.

As used herein, "cell membrane" preparation refers to a preparation of cellular lipid membranes and is distinct from a cellular homogenate, in that at least a portion (i.e., at least 10%, and preferably more) of non-membrane-associated cellular constituents have been removed from the homogenate. "Membrane associated" refers to a polypeptide that is either integrated into a lipid membrane or is physically associated with a component that is integrated into a lipid membrane.

The terms "polypeptide" and "protein" are generally used interchangeably and refer to a single polypeptide chain which may or may not be modified by addition of non-amino acid groups. It would be understood that such polypeptide chains may associate with other polypeptides or proteins or other molecules such as co-factors. The terms "proteins" and "polypeptides" as used herein also include variants, mutants, biologically active fragments, modifications, analogous and/or derivatives of the polypeptides described herein.

The % identity of a polypeptide is determined by GAP analysis (GCG program) with a gap creation penalty of 5, and a gap extension penalty of 0.3. The query sequence is at least 25 amino acids in length, and the GAP analysis aligns the two sequences over a region of at least 25 amino acids. More preferably, the query sequence is at least 50 amino acids in length, and the GAP analysis aligns the two sequences over a region of at least 50 amino acids. More preferably, the query sequence is at least 100 amino acids in length and the GAP analysis aligns the two sequences over a region of at least 100 amino acids. Even more preferably, the query sequence is at least 250 amino acids in length and the GAP analysis aligns the two sequences over a region of at least 250 amino acids. Even more preferably, the GAP analysis aligns the two sequences over their entire length.

As used herein, a "biologically active fragment" is a portion of a polypeptide as described herein, which maintains a defined activity of the full-length polypeptide. For example, a biologically active fragment of a voltage-dependent ion subunit must be capable of binding the target compound, resulting in a conformational change. Biologically active fragments can be any size as long as they maintain the defined activity. Preferably, biologically active fragments are at least 150, more preferably at least 250 amino acids in length.

As used herein, a "biologically active variant" is a molecule which differs from a naturally occurring and/or defined molecule by one or more amino acids but maintains a defined activity, such as defined above for biologically active fragments. Biologically active variants are typically least 50%, more preferably at least 80%, more preferably at least 90%, more preferably at least 95%, more preferably at least 97%, and even more preferably at least 99% identical to the naturally occurring and/or defined molecule.

With regard to a defined polypeptide or polynucleotide, it will be appreciated that % identity figures higher than those provided above will encompass preferred embodiments. Thus, where applicable, in light of the minimum % identity figures, it is preferred that the polypeptide or polynucleotide comprises an amino acid sequence which is at least 50%, more preferably at least 60%, more preferably at least 70%, more preferably at least 80%, more preferably at least 85%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, and even more preferably at least 99.9% identical to the relevant nominated amino acid sequences.

By an "isolated polynucleotide", including DNA, RNA, or a combination of these, single or double stranded, in the sense or antisense orientation or a combination of both, we mean a polynucleotide which is at least partially separated from the polynucleotide sequences with which it is associated or linked in its native state. Preferably, the isolated polynucleotide is at least 60% free, preferably at least 75% free, and most preferably at least 90% free from other components with which they are naturally associated.

As used herein, the terms "determining," "measuring," "assessing," and "assaying" are used interchangeably and include both quantitative and qualitative determinations.

As used herein, "recombinant vector" refers to a discrete genetic element that is used to introduce heterologous nucleic acid into cells for either expression or replication thereof. Selection and use of such vehicles are well within the skill of the artisan. A recombinant expression vector includes vectors capable of expressing nucleic acids that are operatively linked to regulatory sequences, such as promoter regions that are capable of effecting expression of such nucleic acids. Thus, an expression vector refers to a recombinant DNA or RNA construct, such as a plasmid, a phage, recombinant virus or other vector that, upon introduction into an appropriate host cell, results in expression of the cloned nucleic acid. Appropriate expression vectors are well known to those of skill in the art and include those that are replicable in eukaryotic cells and/or prokaryotic cells including those that remain episomal or those, which integrate into the host cell genome.

The terms "ion conducting channel" are intended to refer to ion-permeable pores in the lipid membranes of all cells, which are known to open and close in response to stimuli, gating the flow of specific small ions. Indeed, the lipid bilayer membrane of all cells forms a barrier that is largely impermeable to the flux of ions and water. Residing within the membrane is a family of proteins called ion channels or ion conducting channels, which provide selective pathways for ion flux. Such ion conducting channels are thus ion-selective channels, for example sodium-, potassium, calcium-, chloride- selective or non-selective channels.

Flux of ions through ion conducting channels contributes to depolarization of cells causing the membrane potential to become more positive and generating cytoplasmic regulatory signaling. For example, regulated conductance produced by ion channels are required for intercellular signaling and neuronal excitability. We may cite a group of ion channels that open upon depolarization of excitable cells are classified as voltage-gated and are responsible for electrical activity in nerve, muscle and cardiac tissue. For example, in neurons, ion currents flowing through voltage-gated sodium ion (Na⁺) channels are responsible for rapid spike-like action potentials. During action potentials the majority of Na⁺ channels open very briefly. These brief openings result in transient Na⁺ currents. However, a subset of voltage-gated Na⁺ channels does not close rapidly but remain open for relatively long intervals. These channels therefore generate sustained or persistent Na⁺ currents. The balance between transient and persistent Na⁺ current is crucial for maintaining normal physiological function and electrical signaling throughout the entire nervous system.

Over the past 5 decades, an increasing number of diseases have been shown to result from the dysregulation of ion conducting channels. This class of disease has been termed channelopathies. Aberrant persistent sodium current can contribute to the development or progression of many channelopathies because normal function is disrupted when neurons discharge signals inappropriately. For example, abnormal persistent sodium current is thought to induce deleterious phenomena, including, e.g., neuropathies, neurodegenerative diseases, movement disorders, cardiac arrhythmia, epileptic seizure, neuronal cell death, behavioral disorders and dementia etc... For example, in the case of the neuropathies embraced by epilepsy, there can be a brief electrical "storm" arising from neurons that are inherently unstable because of a genetic defect as in various types of inherited epilepsy, or from neurons made unstable by metabolic abnormalities such as low blood glucose, or alcohol. In other cases, the abnormal discharge can come from a localized area of the brain, such as in patients with epilepsy caused by head injury or brain tumor. In the case of ischemic injuries, such as, e.g., cerebral ischemia and myocardial ischemia, there can be prolonged electrical activity arising from neurons in which persistent sodium channel expression or activity is increased. Such aberrant electrical activity can cause or contribute to neuronal death, which can lead to debilitating injury or death of an individual. Aberrant electrical activity also can contribute to neurodegenerative disorders such as, without limitation, Parkinson's disease, Alzheimer's disease, Huntington's disease, amyotrophic lateral sclerosis and multiple sclerosis. Thus, aberrant persistent sodium current can contribute to development or progression of pathological conditions by collapsing the normal cell transmembrane gradient for sodium, leading to reverse operation of the sodium-calcium exchanger, and resulting in an influx of intracellular calcium, which injures the axon. Therefore, selective reduction in the expression or activity of sodium channels capable of mediating persistent current relative to any reduction in normal voltage-gated (transient) sodium current can be useful for treating channelopathies associated with increased persistent sodium current.

Thus, there exists a need for new screening methods that can be used to identify persistent ion channel modulators either inhibitors or activators useful for treating channelopathies. The present invention satisfies this need by providing high-throughput screens for identifying ion channel modulators, activators or inhibitors.

One technique for assessing protein-protein interaction is based on fluorescence resonance energy transfer (FRET). FRET is a proximity assay based on the non-radiative transfer of energy *i.e.,* a transfer of energy which occurs without emission of photons, and which results in a dipole-dipole interaction between a donor of energy and an acceptor of energy. In FRET, one excited state fluorophore donor transfers its energy to a ground state fluorescent acceptor molecule that become activated. Following activation, the acceptor returns at basal stage by emitting a photon at its own wavelength. Energy transfer efficiency is dependent on the distance between the donor and acceptor, the extent of the spectral overlap and the relative orientation of the acceptor and donor dipoles.

Experiments on the neuronal voltage-gated N-type (Cav2.2) calcium using a FRET-based calcium biosensor named TN-XL have been conducted in the past (See Tay et al., (Nature Communications, Vol, 3; 778, 2012). The authors explored whether troponin-based TN-XL, an optical, genetically encoded calcium ion indicator, could respond to Ca²⁺ when targeted to this environment using a specific approach of fusing TN-XL to the carboxy terminus of the principle subunit of CaV2.2 Ca²⁺ channels. These experiments failed to provide any reliable and useful means for measuring the activity of an ion conducting channel. Indeed, in order to gauge TN-XL responsiveness, authors had to measure using TIRF imaging, the single-cell CFP and FRET fluorescence signals where the ratio of these signals furnishes the customary sensor readout of Ca²⁺. In addition, in order to improve the TN-XL signal-to-noise ratio at the surface membrane, the authors had to utilize TIRF microscopy to selectively illuminate only those CaV2.2/ TN-XL channels near the surface, thereby attenuating the background signal from imperfectly targeted intracellular channels. Further in order to facilitate elevation of intracellular Ca²⁺, additional steps like internal Ca2 + buffering (1 mM EGTA) with elevated 10 mM extracellular Ca²⁺ was employed. Further limitation of the study concerned the incomplete targeting of active CaV2.2/TN-XL channels to the surface membrane in the TIRF volume, necessitating approximate correction of static background fluorescence. The potential error in such correction rendered the calcium spike determination as a coarse approximate estimate, accordingly such a technique is not at all suitable for HTS.

Accordingly, this study clearly revealed the complexity and difficulties encountered when using the FRET-based calcium biosensor, requiring a complex integration of TIRF/patch-clamp electrophysiology and CaV2.2/TN-XL fusion, instead of a straightforward and simple method based on the fusion construct alone.

Alternative to FRET technique is the BRET technique. BRET method is similar to that of FRET wherein the donor is a bioluminescent molecule. No external source of excitation is thus required, thereby avoiding inconveniences of photobleaching and autofluorescence of the cells/tissues. Typically, the bioluminescent donor used may be luciferase and the acceptor may be any suitable fluorophore similar to that of FRET. The use of a luciferase avoids the need for illumination as the addition of a substrate initiates bioluminescent emission and thus resulting to resonance energy transfer between a bioluminescent donor and a fluorescent acceptor. Both FRET and BRET transfer efficiency also depends on the degree of the spectral overlap or partial spectral overlap between the donor and acceptor, the relative orientation between the emission dipole of the donor and the absorption dipole of the acceptor, and the distance between the donor and acceptor, and further by the efficacy of the ion sensor placed strategically between the donor and acceptor in the novel fusion construct. It would thus have been expected to one skilled person in the art that similar BRET-based ion biosensor would have provided similar results and thus could not be exploitable for drug discovery or HTS method.

In contrast with previous teaching and experimentations with FRET, the present applicant surprisingly showed for the first time that it was possible to construct and use BRET-based ion sensor in order to assess flux of ions and thus actual activation or inhibition of ion conducting channels in an accurate and reliable manner. The applicant showed that the new BRET probe may be used in a screening system that targets ion conducting channels with superior efficiency and particularly for HTS method without any requirements of further analysis such as for patch-clamp experiments, etc...

The present invention thus relates to novel ion conducting channel protein subunit fusions comprising an ion conducting channel subunit bound to a probe, which probe comprises an ion sensor bound in between or "sandwiched" between at least one bioluminescent donor molecule and at least one fluorescent acceptor molecule. The probe may be bound to the channel subunit, optionally via a linker, at its C-terminal, its N-terminal end, or within an intracellular or extracellular loop, either the bioluminescent donor molecule or the fluorescent acceptor molecule. The ion sensor is configured to undergo a conformational change in the presence of an ion transported by the channel subunit. The bioluminescent donor molecule and fluorescent acceptor molecule are selected so that the emission spectrum of the bioluminescent donor molecule overlaps with the absorbance spectrum of the acceptor molecule, making it possible the non-radiative energy transfer between the bioluminescent energy donor and the fluorescent acceptor through nonradiative dipole-dipole coupling. The novel channel protein fusion subunits may thus be used as novel BRET probes for detecting activation, inhibition or modulation of ion conducting channels by measuring the actual passages of ions through said channels.

The present invention also relates to novel ion conducting channel fusions which result from the assembling of the fusion subunits as described above. Therefore, such novel ion conducting channel protein fusion may comprise one or more of its subunits bound either in C-terminal, in N-terminal, or within an intracellular or extracellular loop of the subunit to a probe which itself comprises an ion sensor being sandwiched between at least one bioluminescent donor molecule and at least one fluorescent acceptor molecule. The ion sensor is configured to undergo a conformational change in the presence of an ion transported by the channel fusion. Preferably, all subunits of the ion conducting channel protein fusion may be channel subunit fusions. As indicated above, the bioluminescent donor molecule and fluorescent acceptor molecule are selected so that the emission spectrum of the bioluminescent donor molecule overlaps with the absorbance spectrum of the acceptor molecule, making it possible the non-radiative energy transfer between the bioluminescent energy donor and the fluorescent acceptor through nonradiative dipole-dipole coupling. The novel channel protein fusions may thus be used as novel BRET probes for detecting activation, inhibition or modulation of ion conducting channels by measuring the actual passages of ions through said channels.

The present invention also relates to a nucleotide sequence encoding a channel fusion subunit comprising a subunit of an ion conducting channel bound to a probe which itself probe comprises an ion sensor bound in between to at least a bioluminescent donor molecule and to at least a protein fluorescent acceptor molecule. As described above, the ion sensor is configured to undergo a conformational change in the presence of an ion transported by the channel subunit, and the probe is bound to the N-terminal or the C-terminal, or within an intracellular or extracellular loop of said channel subunit via either the bioluminescent donor molecule or the protein fluorescent acceptor molecule. The nucleotide sequence or construct according to the present invention may optionally further comprise a linker sequence between the nucleotide sequence encoding the ion conducting channel subunit and the nucleotide sequence encoding the probe.

All the known ion conducting channels are within the scope of the present invention as most ion-channels have such common three-dimensional structure. Typically, these ion conducting channels at least have three functional domains: a first domain corresponding to the ion conducting pore, *i.e.,* a selectivity filter that distinguishes among the ions that do go through and the ions that do not; a second domain corresponding to the gate, *i.e.,* a part of the channel that can open and close the conducting pore; and a third domain corresponding to the detector of stimuli that responds to electrical potential changes or chemical signals. The detectors of stimuli thus typically couple to the channel gates to control the probability that they open or close the pores.

The channel is formed by subunits that each cross the plasma membrane at least twice. Various subunits are assembled together to form a channel. Whether the channel is formed by homomeric assembly of the same subunits or the heteromeric combination of an alpha subunit and other subunits heteromeric or homomeric subunits depends on the channel type. In the center of the assembled channel is the pore, a narrow opening that allows ions such as Na⁺, K⁺, Ca²⁺, and/or Cl⁻ to flow across the membrane. The pore is responsible for the permeability to the selected ion(s).

Ion conducting channel are thus made up of several subunits which are organized in the plasma membrane to create a pore through which cations or anions can move. Ion conducting channels may be classified by gating, *i.e.,* what opens and closes the channels, and may include in particular voltage-dependent ion channels or ligand-dependent ion channel. Voltage-gated ion channels activate/inactivate depending on the voltage gradient across the plasma membrane, while ligand-gated ion channels activate/inactivate depending on binding of ligands to the channel.

Voltage-dependent ion channels are membrane proteins that conduct ions at high rates regulated by the voltage across the membrane. These channel proteins are thus selective for particular ions, such as for example, calcium, potassium, sodium, or chloride ions. Most studied and well-known are voltage-gated Na⁺, K⁺, and Ca²⁺ channels. They generally share several common tetrameric structural configuration formed by four homologous or heterologous subunits with a central pore with highly conserved pore-lining residues for channels with similar charge selectivity; common gating strategy using a charged membrane voltage sensor; and auxiliary subunits that regulate channel protein trafficking and function. For example, channels are made up of four heterologous subunits include K+ channels, or channels that are made of four homologous domains include Na+ and Ca2+ channels. Each of the subunits or domains has six transmembrane segments and a pore loop. The fifth and sixth transmembrane segments (S5 and S6) and the pore loop are responsible for ion conduction. Prominent among these are the voltage-gated Na+, K+, and Ca2+ channels that underlie the action potentials and the rapid calcium signaling of electrically excitable cells. Fast voltage-dependent opening of Na+ channels accounts for all-or-nothing excitation initiated with a sharp threshold by depolarizing stimuli and for the regenerative spread of excitation as an action potential propagates along an axon or muscle fiber.

Some are at best weakly voltage dependent, although they retain voltage-sensor domains with a few positive charges. Included among these are the cyclic nucleotide gated (CNG) and the TRP families of non-selective cation channels. The CNG channels serve vertebrate phototransduction and olfaction. The TRP family includes diverse forms serving in phototransduction of invertebrates and in sensory receptors detecting hot, cold, chili peppers, mustard, ginger, and possibly touch, pressure, and motion. Another family of ion channels, the inwardly rectifying K⁺ channels, has no voltage-sensor domain but has a pore-forming domain with much sequence identity and ion selectivity identical to that of voltage-gated K⁺ channels.

Voltage-dependent ion channels are a proven target for drug discovery, and many ion channel modulators are currently in clinical use for the treatment of pain, epilepsy, hypertension and other disease states. They comprise the molecular basis for essential physiological functions including fluid secretion, electrolyte balance, and bioenergetics and membrane excitability.

Voltage-dependent ion channels are known to exist in several different conformational states, referred to as gating states. A voltage-gated ion channel can be viewed as residing in one of 3 gating states-closed (no ion permeation), open (ion flux occurs) and inactivated (no ion permeation; channel cannot be opened by depolarization), although it should be noted that some channels do not exhibit an inactivated state. Transition between gating states is voltage-dependent, and at any given time, equilibrium exists between these gating states, with the proportion of channels residing in each state depending upon the cellular membrane potential. Many voltage-dependent ion channel modulators have been shown to bind preferentially to a specific gating state or states. For example, the voltage-gated sodium channel blocker Lamotrigine is thought to bind to the opened and inactivated states of the brain sodium channel protein. Preferential binding to a particular gating state may occur through an increase in channel affinity for the ion channel modulator, or simply through improved access of the drug to its binding site on the channel.

A complete account of the voltage-gated ion channels that are within the scope of the present invention, are tabulated below.

**Table 1: Sodium channels, voltage-gated**

| Approved Symbol | Approved Name | Tissue distribution | Synonyms | Chromosome | Accession numbers on uniprot.org website |
|---|---|---|---|---|---|
| SCN1A | sodium channel, voltage-gated, type I, alpha subunit | CNS | Nav1.1, GEFSP2, HBSCI, NAC1, SMEI | 2q24.3 | **P35498** https://www.uniprot.org/uniprot/ P35498 |
| | | | | | E9PG49 Q9C008, X03638 X65362, AF003372 |
| SCN1B | sodium channel, voltage-gated, type I, beta subunit | CNS- brain, skeletal muscle, and heart | | 19q13.12 | **Q07699** https://www.uniprot.org/uniprot/ Q07699 |
| | | | | | Q5TZZ4, Q6TN97, M91808, L10338 |
| SCN2A | sodium channel, voltage-gated, type II, alpha subunit | CNS | Nav1.2, HBSCII, HBSCI | 2q24.3 | **Q99250,** https://www.uniprot.org/uniprot/ Q99250 |
| | | | | | A6NC14, A6NC14, X03639, X65361, M94055, X61149 |
| SCN2B | sodium channel, voltage-gated, type II, beta subunit | CNS | | 11q23.3 | **O60939** https://www.uniprot.org/uniprot/ O60939 |
| | | | | | O75302, Q9UNN3, U37026, U37147, AF007783 |
| SCN3A | sodium channel, voltage-gated, type III, alpha subunit | CNS | Nav1.3 | 2q24 | **Q9NY46** https://www.uniprot.org/uniprot/ Q9NY46 |
| | | | | | Q16142 Q9Y6P4, Y00766 |
| SCN3B | sodium channel, voltage-gated, type III, beta subunit | Heart | HSA243396 | 11q24.1 | **Q9NY72** https://www.uniprot.org/uniprot/ Q9NY72 |
| SCN4A | sodium channel, voltage-gated, type IV, alpha subunit | skeletal muscle-denervated and innervated skeletal muscle | Nav1.4, HYPP, SkM1 | 17q23.3 | **P35499** https://www.uniprot.org/uniprot/ P35499 |
| | | | | | Q15478, Q16447, Q7Z6B1 M26643, M81758 |
| SCN5A | sodium channel, voltage-gated, type V, alpha subunit | denervated skeletal muscle, heart | Nav1.5, LQT3, HB1, HBBD, PFHB1, IVF, HB2, HH1, SSS1, CDCD2, CMPD2, ICCD | 3p21 | **Q14524** https://www.uniprot.org/uniprot/ Q14524 |
| | | | | | M27902, M77235, **Q86V90,** A5H1P8, A5H1P8 |
| SCN7A | sodium channel, voltage-gated, type VII, alpha subunit | astrocytes PNS (DRG) | Nav2.1, Nav2.2, NaG | 2q21-q23 | **Q01118** https://www.uniprot.org/uniprot/ Q01118 |
| | | | | | M96578 Y09164 |
| SCN8A | sodium channel, voltage gated, type VIII, alpha subunit | CNS | Nav1.6, NaCh6, PN4, Cerlll | 12q 13.1 | **Q9UQD0** https://www.uniprot.org/uniprot/ Q9UQD0 |
| | | | | | L39018 AF049239A F049240 U26707 AF049617 AF050736 AF003373 , Q9UPB2, B9VWG8 |
| SCN9A | sodium channel, voltage-gated, type IX, alpha subunit | medullary thyroid Ca Schwann Cells PNS | Nav1.7, PN1, NE-NA, NENA, ETHA | 2q24 | **Q15858** https://www.uniprot.org/uniprot/ Q15858 |
| | | | | | A1BUH5, Q8WWN4,U79568 X82835, U35238 |
| SCN10A | sodium channel, voltage-gated, type X, alpha subunit | PNS | Nav1.8, hPN3, SNS, PN3 | 3p22.2 | **Q9Y5Y9** https://www.uniprot.org/uniprot/ Q9Y5Y9 |
| | | | | | A6NDQ1, X92184, U53833 Y09108 |
| SCN11A | sodium channel, voltage-gated, type XI, alpha subunit | | Nav1.9, NaN, SNS-2 | 3p22.2 | **Q9UI33** https://www.uniprot.org/uniprot/ Q9UI33 |
| | | | | | A6NN05, Q9UHM0 |

**Table 2: Calcium channels, voltage-gated**

| Approved Symbol | Approved Name | Tissue distribution | Synonyms | Chromosome | Accession numbers on uniprot.org website |
|---|---|---|---|---|---|
| CACNA1A | calcium channel, voltage-dependent, P/Q type, alpha 1A subunit | Brain specific; mainly found in cerebellum, cerebral cortex, thalamus and hypothalamus. Expressed in the small cell lung carcinoma cell line SCC-9 | Cav2.1, EA2, APCA, HPCA, FHM | 19p13 | **O00555** https://www.uniprot.org/unip rot/O00555 J3KP41, Q9UDC4 |
| CACNA1B | calcium channel, voltage-dependent, N type, alpha 1B subunit | Isoform Alpha-1 b-1 and isoform Alpha-1b-2 are expressed in the central nervous system | Cav2.2, CACNN | 9q34 | Q00975 https://www.uniprot.org/unip rot/Q00975 B1AQK5 |
| CACNA1C | calcium channel, voltage-dependent, L type, alpha 1C subunit | Expressed in brain, heart, jejunum, ovary, pancreatic beta-cells and vascular smooth muscle. Overall expression is reduced in atherosclerotic vascular smooth muscle | Cav1.2, CACH2, CACN2, TS, LQT8 | 12p13.3 | **Q13936** https://www.uniprot.org/unip rot/Q13936 B2RUT3, Q99875 |
| CACNA1D | calcium channel, voltage-dependent, L type, alpha 1D subunit | Expressed in pancreatic islets and in brain, where it has been seen in cerebral cortex, hippocampus, basal ganglia, habenula and thalamus. Expressed in the small cell lung carcinoma cell line SCC-9. | Cav1.3, CACH3, CACN4 | 3p14.3 | **Q01668** https://www.uniprot.org/unip rot/Q01668 B0FYA3, Q9UDC3 |
| CACNA1E | calcium channel, voltage-dependent, R type, alpha 1E subunit | Expressed in neuronal tissues and in kidney | Cav2.3, Bll, CACH6 | 1q25.3 | **Q15878** https://www.uniprot.org/unip rot/Q15878 Q14581, B1AM12 |
| CACNA1F | calcium channel, voltage-dependent, L type, alpha 1F subunit | Expression in skeletal muscle and retina | Cav1.4, JM8, JMC8, CSNBX2, CORDX3, CSNB2A, OA2 | Xp11.23 | **O60840** https://www.uniprot.org/unip rot/O60840 A6NI29, Q9UHB1 |
| CACNA1G | calcium channel, voltage-dependent, T | Highly expressed in brain, in particular in the amygdala, subthalamic nuclei, | Cav3.1, NBR13 | 17q22 | **O43497** https://www.uniprot.org/unip rot/O43497 D6RA64, Q9Y5T3 |
| | type, alpha 1G subunit | cerebellum and thalamus. Moderate expression in heart; low expression in placenta, kidney and lung. Also expressed in colon and bone marrow and in tumoral cells to a lesser extent. Highly expressed in fetal brain, but also in peripheral fetal tissues as heart, kidney and lung, suggesting a developmentally regulated expression | | | |
| CACNA1H | calcium channel, voltage-dependent, T type, alpha 1H subunit | Expressed in kidney, liver, heart brain. Isoform 2 seems to be testis-specific | Cav3.2 | 16p13.3 | **O95180** https://www.uniprot.org/unip rot/O95180 B5ME00, Q9NYY5 |
| CACNA1I | calcium channel, voltage-dependent, T type, alpha 11l subunit | Brain specific | Cav3.3 | 22q13.1 | **Q9P0X4** https://www.uniprot.org/unip rot/Q9P0X4 B0QY12, Q9UNE6 |
| CACNA1S | calcium channel, voltage-dependent, L type, alpha 1S subunit | Skeletal muscle specific. | Cav1.1, hypoPP | 1q32 | **Q13698** https://www.uniprot.org/unip rot/Q13698 A4IF51, Q13934 |

**Table 3: Transient receptor potential cation channels**

| Approved Symbol | Approved Name | Tissue distribution | Synonyms | Chromosome | Accession numbers on uniprot.org website |
|---|---|---|---|---|---|
| TRPA1 | transient receptor potential cation channel, subfamily A, member 1 | Expressed at very low level in human fibroblasts and at a moderate level in liposarcoma cells | | 8q13 | **O75762** https://www.uniprot.org/unip rot/O75762 A6NIN6 |
| TRPC1 | transient receptor potential cation channel, subfamily C, member 1 | Seems to be ubiquitous | HTRP-1 | 3q23 | **P48995** https://www.uniprot.org/unip rot/P48995 Q14CE4 |
| TRPC2 | transient receptor potential cation channel, subfamily C, member 2, pseudogene | | | 11p15.4 | **Q6ZNB5** |
| TRPC3 | transient receptor potential cation channel, subfamily C, member 3 | Expressed predominantly in brain and at much lower levels in ovary, colon, small intestine, lung, prostate, placenta and testis | | 4q27 | **Q13507**, A7VJS1, Q5G1L5 |
| TRPC4 | transient receptor potential cation channel, subfamily C, member 4 | Strongly expressed in placenta. Expressed at lower levels in heart, pancreas, kidney and brain. Expressed in endothelial cells. Isoform alpha was found to be the predominant isoform. | HTRP4, TRP4 | 13q13.3 | **Q9UBN4,** B1ALE0, Q9UIB2 |
| TRPC5 | transient receptor potential cation channel, subfamily C, member 5 | Expressed in brain with higher levels in fetal brain. Found in cerebellum and occipital pole | PPP1R159 | Xq23 | **Q9UL62**, B2RP53 Q9Y514 |
| TRPC6 | transient receptor potential cation channel, subfamily C, member 6 | Expressed primarily in placenta, lung, spleen, ovary and small intestine. Expressed in podocytes and is a component of the glomerular slit diaphragm | TRP6 | 11q22.1 | **Q9Y210** Q52M59 Q9NQA9 |
| TRPC7 | transient receptor potential cation channel, subfamily C, member 7 | Highly expressed in brain and peripheral blood cells, such as neutrophils. Also detected in bone marrow, spleen, heart, liver and lung. Isoform 2 is found in neutrophil granulocytes | | 5q31.2 | **O94759** D3DSL6 Q96Q93_**Q9HCX4** A1A4Z4 Q8IWP7 |
| TRPM1 | transient receptor potential cation channel, subfamily M, member 1 | Expressed in the retina where it localizes to the outer plexiform layer. Highly expressed in benign melanocytic nevi and diffusely expressed in various in situ melanomas, but not detected in melanoma metastases. Also expressed in melanocytes and pigmented metastatic melanoma cell lines. In melanocytes expression appears to be regulated at the level of transcription and mRNA processing | L TRPC1, CSNB1C | 15q13.3 | **Q7Z4N2** D9IDV2 Q7Z4N5 |
| TRPM2 | transient receptor potential cation channel, subfamily M, member 2 | Detected in blood plasma, cerebrospinal fluid, milk, seminal plasma and colon mucosa. Detected in the germinal center of colon lymphoid nodules and in colon parasympathetic ganglia of the Auerbach plexus (at protein level). Ubiquitous. Detected in brain, testis, ovary, liver and pancreas, and at lower levels in kidney, heart, spleen and lung | KNP3, LTRPC2, NUDT9L1, NUDT9H, EREG1 | 21q22.3 | **P10909** B2R9Q1 Q7Z5B9 |
| TRPM3 | transient receptor potential cation channel, subfamily M, member 3 | | KIAA1616, LTRPC3, GON-2 | 9q21.11 | **Q504Y1** |
| TRPM4 | transient receptor potential cation channel, subfamily M, member 4 | Widely expressed with a high expression in intestine and prostate. In brain, it is both expressed in whole cerebral arteries and isolated vascular smooth muscle cells. Prominently expressed in Purkinje fibers. Expressed at higher levels in T-helper 2 (Th2) cells as compared to T-helper 1 (Th1) cells | FLJ20041 | 19q13.3 | **Q8TD43** A2RU25 Q9NXV1 |
| TRPM5 | transient receptor potential cation channel, subfamily M, member 5 | Strongly expressed in fetal brain, liver and kidney, and in adult prostate, testis, ovary, colon and peripheral blood leukocytes. Also expressed in a large proportion of Wilms' tumors and rhabdomyosarcomas. In monochromosomal cell lines shows exclusive paternal expression | LTRPC5, MTR1 | 11p15.5 | **Q9NZQ8** A6NHS0, Q52LU2, Q9NY34 |
| TRPM6 | transient receptor potential cation channel, subfamily M, member 6 | Highly expressed in kidney and colon. Isoform TRPM6a and isoform TRPM6b, are coexpressed with TRPM7 in kidney, and testis, and are also found in several cell lines of lung origin. Isoform TRPM6c is detected only in testis and in NCI-H510A small cell lung carcinoma cells. | CHAK2, FLJ22628 | 9q21.13 | **Q9BX84** Q6VPR8 Q6VPS2 |
| TRPM7 | transient receptor potential cation channel, subfamily M, member 7 | | CHAK1, LTRPC7, TRP-PLIK | 15q21 | **Q96QT4** Q6ZMF5 Q9NXQ2 |
| TRPM8 | transient receptor potential cation channel, subfamily M, member 8 | Expressed in prostate. Also expressed in prostate tumors and in non-prostatic primary tumors such as colon, lung, breast and skin tumors | | 2q37 | **Q7Z2W7,** A0AVG2, Q9BVK1 |
| MCOLN1 | mucolipin 1 | Widely expressed in adult and fetal tissues | TRPML1, ML4, MLIV, MST080, MSTP080, TRPM-L1 | 19p13.2 | **Q9GZU1** D6W647 Q9H4B5 |
| MCOLN2 | mucolipin 2 | | TRPML2, FLJ36691, TRP-ML2 | 1p22 | **Q8IZK6** A6NI99 Q8N9R3 |
| MCOLN3 | mucolipin 3 | | TRPML3, FLJ11006, TRP-ML3 | 1p22.3 | **Q8TDD5** Q5T4H5, Q5T4H6, Q9NV09 |
| PKD1 | polycystic kidney disease 1 (autosomal dominant) | | PBP, Pc-1, TRPP1 | 16p13.3 | **P98161** Q15140 Q15141 |
| PKD2 | polycystic kidney disease 2 (autosomal dominant) | Strongly expressed in ovary, fetal and adult kidney, testis, and small intestine. Not detected in peripheral leukocytes | PKD4, PC2, Pc-2, TRPP2 | 4q22.1 | **Q13563** 060441 Q2M1Q5 |
| PKD2L1 | polycystic kidney disease 2-like 1 | Expressed in adult heart, skeletal muscle, brain, spleen, testis, retina and liver. | PCL, TRPP3 | 10q24.31 | **Q9P0L9** O75972 Q9UPA2 |
| PKD2L2 | polycystic kidney disease 2-like 2 | Testis, Brain and Kidney | TRPP5 | 5q31 | **Q9NZM6** A6NK98 Q9UNJ0 |
| TRPV1 | transient receptor potential cation channel, subfamily V, member 1 | Widely expressed at low levels. Expression is elevated in dorsal root ganglia. In skin, expressed in cutaneous sensory nerve fibers, mast cells, epidermal keratinocytes, dermal blood vessels, the inner root sheet and the infundibulum of hair follicles, differentiated sebocytes, sweat gland ducts, and the secretory portion of eccrine sweat glands (at protein level). | | 17p13.2 | **Q8NER1** A2RUA9 Q9NY22 |
| TRPV2 | transient receptor potential cation channel, subfamily V, member 2 | | VRL, VRL-1, VRL1 | 17p11.2 | **Q9Y5S1,** A6NML2, A8K0Z0, Q9Y670 |
| TRPV3 | transient receptor potential cation channel, subfamily V, member 3 | Abundantly expressed in CNS. Widely expressed at low levels. Detected in dorsal root ganglion (at protein level). Expressed in the keratinocyte layers of the outer root sheath and, to lesser extent, to the matrix of the hair follicles (at protein level). | VRL3 | 17p13.3 | **Q8NET8,** Q8NDW7, Q8NET9, Q8NFH2 |
| TRPV4 | transient receptor potential cation channel, subfamily V, member 4 | Found in the synoviocytes from patients with (RA) and without (CTR) rheumatoid arthritis (at protein level). | OTRPC4, TRP12, VROAC, VRL-2, VROAC, CMT2C | 12q24.1 | **Q9HBA0,** B7ZKQ6,Q9HBC0 |
| TRPV5 | transient receptor potential cation channel, subfamily V, member 5 | Expressed at high levels in kidney, small intestine and pancreas, and at lower levels in testis, prostate, placenta, brain, colon and rectum | CaT2 | 7q34 | **Q9NQA5,** A4D2H7, Q96PM6 |
| TRPV6 | transient receptor potential cation channel, subfamily V, member 6 | Expressed at high levels in the gastrointestinal tract, including esophagus, stomach, duodenum, jejunum, ileum and colon, and in pancreas, placenta, prostate and salivary gland. Expressed at moderate levels in liver, kidney and testis. Expressed in locally advanced prostate cancer, metastatic and androgen-insensitive prostatic lesions but not detected in healthy prostate tissue and benign prostatic hyperplasia | CaT1 | 7q34 | **Q9H1D0,** A4D2I8, Q9H296 |

**Table 4: CatSper channels**

| Approved Symbol | Approved Name | Tissue distribution | Synonyms | Chromosome | Accession numbers on uniprot.org website |
|---|---|---|---|---|---|
| CATSPER1 | cation channel, sperm associated 1 | Testis-specific | CATSPER | 11q12.1 | **Q8NEC5** Q96P76 |
| CATSPER2 | cation channel, sperm associated 2 | Testis-specific | | 15q15.3 | **Q96P56,** Q8NHT9, Q96P54, Q96P55 |
| CATSPER3 | cation channel, sperm associated 3 | Testis-specific | CACRC | 5q31.2 | **Q86XQ3,** Q86XS6 |
| CATSPER4 | cation channel, sperm associated 4 | Testis-specific | | 1p35.3 | **Q7RTX7,** A1A4W6, Q5VY71 |

**Table 5: Two-pore channels**

| Approved Symbol | Approved Name | Tissue distribution | Synonyms | Chromosome | Accession numbers on uniprot.org website |
|---|---|---|---|---|---|
| TPCN1 | two pore segment channel 1 | | KIAA1169, FLJ20612, TPC1 | 12q24.21 | **B7Z3R2**_ **A5PKY2_ H0YIK4 F8VV93** |
| TPCN2 | two pore segment channel 2 | Widely expressed. Expressed at high level in liver and kidney | TPC2 | 11q13.1 | **Q8NHX9** Q9NT82_**E7ETX0** |

**Table 6: Cyclic nucleotide-regulated channels**

| Approved Symbol | Approved Names | Tissue Distribution | Synonyms | Chromosome | Accession numbers on uniprot.org website |
|---|---|---|---|---|---|
| CNGA1 | cyclic nucleotide gated channel alpha 1 | Rod cells in the retina | RCNC1, RCNCa, CNG1, RP49 | 4p12 | **P29973** A8K7K6 Q4W5E, **D6RCF1_D6R978, A0A024R9X3, Q14028,** H3BN09 Q9UMG2 |
| CNGA2 | cyclic nucleotide gated channel alpha 2 | | CNG2, OCNC1, OCNCa, OCNCALPHA , OCNCalpha, FLJ46312 | Xq27 | **Q16280,**A0AVD0**,Q8IV77** |
| CNGA3 | cyclic nucleotide gated channel alpha 3 | Prominently expressed in retina | CCNC1, CCNCa, CNG3 | 2q11.2 | **Q16281,** Q4VAP7, Q53RD2, Q9UP64 |
| CNGA4 | cyclic nucleotide gated channel alpha 4 | | OCNC2, OCNCb, CNG5 | 11p15.4 | **Q8IV77** |
| CNGB1 | cyclic nucleotide gated channel beta 1 | | RCNC2, RCNCb, GARP, GAR1, CNGB1 B, RP45 | 16q13 | **Q16280,_Q16280** |
| CNGB3 | cyclic nucleotide gated channel beta 3 | Expressed specifically in the retina | | 8q21.3 | **Q9NQW8** C9JA51, Q9NRE9, **H0YAZ4 B9EK43,_Q0QD47** |
| HCN1 | hyperpolarization activated cyclic nucleotide-gated potassium channel 1 | Detected in brain, in particular in amygdala and hippocampus, while expression in caudate nucleus, corpus callosum, substantia nigra, subthalamic nucleus and thalamus is very low or not detectable. Detected at very low levels in muscle and pancreas | BCNG-1, HAC-2 | 5p12 | **O60741,_Q86WJ6** |
| HCN2 | hyperpolarization activated cyclic nucleotide-gated potassium channel 2 | Highly expressed throughout the brain. Detected at low levels in heart. | BCNG-2, HAC-1 | 19p13 | **Q9UL51**, 060742, Q9UBS2 |
| HCN3 | hyperpolarization activated cyclic nucleotide-gated potassium channel 3 | Detected in brain | KIAA1535 | 1q21.2 | **Q9P1Z3,** D3DV90, Q9BWQ2,**_Q2T9L6 Q86WJ5** |
| HCN4 | hyperpolarization activated cyclic nucleotide-gated potassium channel 4 | Highly expressed in thalamus, testis and in heart, both in ventricle and atrium. Detected at much lower levels in amygdala, substantia nigra, cerebellum and hippocampus | | 15q24.1 | **Q9Y3Q4** Q9UMQ7 |

**Table 7: Potassium channels, calcium-activated**

| Approved Symbol | Approved Name | Tissue distribution | Synonyms | Chromosome | Accession numbers on uniprot.org website |
|---|---|---|---|---|---|
| KCNMA1 | potassium large conductance calcium-activated channel, subfamily M, alpha member 1 | Widely expressed. Except in myocytes, it is almost ubiquitously expressed | KCa1.1, mSLO1 | 10q22 | **Q12791** F8WA96 Q9UQK6 |
| KCNN1 | potassium intermediate/small conductance calcium-activated channel, subfamily N, member 1 | | KCa2.1, hSK1 | 19p13.1 | **Q92952** Q5KR10, Q6DJU4 |
| KCNN2 | potassium intermediate/small conductance calcium-activated channel, subfamily N, member 2 | Expressed in atrial myocytes (at protein level). Widely expressed | KCa2.2, hSK2 | 5q22.3 | **Q9H2S1** A6NF94 Q6X2Y2 |
| KCNN3 | potassium intermediate/small conductance calcium-activated channel, subfamily N, member 3 | | KCa2.3, hSK3, SKCA3 | 1q 21.3 | **Q9UGI6** B1ANX0 Q8WXG7 |
| KCNN4 | potassium intermediate/small conductance calcium-activated channel, subfamily N, member 4 | Widely expressed in non-excitable tissues | KCa3.1, hSK4, hKCa4, hlKCa1 | 19q13.2 | **015554** Q53XR4 |
| KCNT1 | potassium channel, subfamily T, member 1 | Highest expression in liver, brain and spinal cord. Lowest expression in skeletal muscle | KCa4.1, KIAA1422 | 9q34.3 | **Q5JUK3** B3KXF7, Q9P2C5 |
| KCNT2 | potassium channel, subfamily T, member 2 | | KCa4.2, SLICK, SLO2.1 | 1q31.3 | **Q6UVM3,** Q3SY59, Q5VTN1, Q6ZMT3 |
| KCNU1 | potassium channel, subfamily U, member 1 | Testis-specific | KCa5.1, Slo3, KCNMC1, Kcnma3 | 8p11.2 | **A8MYU2** |

**Table 8: Potassium channels, voltage-gated**

| Approved Symbol | Approved Name | Tissue distribution | Synonyms | Chromosome | Accession numbers on uniprot.org website |
|---|---|---|---|---|---|
| KCNA1 | potassium voltage-gated channel, shaker-related subfamily, member 1 (episodic ataxia with myokymia) | | Kv1.1, RBK1, HUK1, MBK1 | 12p13 | **Q09470** A6NM83, Q3MIQ9 |
| KCNA2 | potassium voltage-gated channel, shaker-related subfamily, member 2 | | Kv1.2, HK4 | 1p13 | **P16389** Q86XG6 |
| KCNA3 | potassium voltage-gated channel, shaker-related subfamily, member 3 | | Kv1.3, MK3, HLK3, HPCN3 | 1p13.3 | **P22001** Q5VWN2 |
| KCNA4 | potassium voltage-gated channel, shaker-related subfamily, member 4 | | Kv1.4, HK1, HPCN2 | 11p14 | **P22459** |
| KCNA5 | potassium voltage-gated channel, shaker-related subfamily, member 5 | Pancreatic islets and insulinoma | Kv1.5, HK2, HPCN1 | 12p13 | **P22460** Q4KKT8 Q9UDA4 |
| KCNA6 | potassium voltage-gated channel, shaker-related subfamily, member 6 | | Kv1.6, HBK2, PPP1 R96 | 12p13 | **P17658** |
| KCNA7 | potassium voltage-gated channel, shaker-related subfamily, member 7 | Highly expressed in skeletal muscle, heart and kidney. | Kv1.7, HAK6 | 19q13.3 | **Q96RP8,** A1KYX7, Q9BYS4 |
| KCNA10 | potassium voltage-gated channel, shaker-related subfamily, member 10 | Detected in kidney, in proximal tubules, glomerular endothelium, in vascular endothelium and in smooth muscle cells | Kv1.8 | 1p13.1 | **Q16322** |
| KCNB1 | potassium voltage-gated channel, Shab-related subfamily, member 1 | | Kv2.1 | 20q13.2 | **Q14721** Q14193 |
| KCNB2 | potassium voltage-gated channel, Shab-related subfamily, member 2 | | Kv2.2 | 8q13.2 | **Q92953,** Q7Z7D0, Q9BXD3 |
| KCNC1 | potassium voltage-gated channel, Shaw-related subfamily, member 1 | | Kv3.1 | 11p15 | **P48547,** K4DI87 |
| KCNC2 | potassium voltage-gated channel, Shaw-related subfamily, member 2 | | Kv3.2 | 12q14.1 | **Q96PR1,** B7Z231, |
| KCNC3 | potassium voltage-gated channel, Shaw-related subfamily, member 3 | | Kv3.3 | 19q13.33 | **Q14003** |
| KCNC4 | potassium voltage-gated channel, Shaw-related subfamily, member 4 | | Kv3.4, HKSHIIIC | 1p21 | **Q03721,** Q3MIM4, Q5TBI6 |
| KCND1 | potassium voltage-gated channel, Shal-related subfamily, member 1 | Widely expressed. Highly expressed in brain, in particular in cerebellum and thalamus; detected at lower levels in the other parts of the brain | Kv4.1 | Xp11.23 | **Q9NSA2** B2RCG0, O75671 |
| KCND2 | potassium voltage-gated channel, Shal-related subfamily, member 2 | Highly expressed throughout the brain. Expression is very low or absent in other tissues. | Kv4.2, RK5, KIAA1044 | 7q31 | **Q9NZV8,** O95012 Q9UNH9 |
| KCND3 | potassium voltage-gated channel, Shal-related subfamily, member 3 | Highly expressed in heart and brain, in particular in cortex, cerebellum, amygdala and caudate nucleus. Detected at lower levels in liver, skeletal muscle, kidney and pancreas. Isoform 1 predominates in most tissues. Isoform 1 and isoform 2 are detected at similar levels in brain, skeletal muscle and pancreas | Kv4.3, KSHIVB | 1p13.2 | **Q9UK17**, 060576, Q9UK16 |
| KCNF1 | potassium voltage-gated channel, subfamily F, member 1 | Detected in heart, brain, liver, skeletal muscle, kidney and pancreas. | Kv5.1, kH1, IK8 | 2p25 | **Q9H3M0**, O43527, Q585L3 |
| KCNG1 | potassium voltage-gated channel, subfamily G, member 1 | Detected in brain and placenta, and at much lower levels in kidney and pancreas | Kv6.1, kH2, K13 | 20q13 | **Q9UIX4,** A8K3S4, Q9BRC1 |
| KCNG2 | potassium voltage-gated channel, subfamily G, member 2 | Highly expressed in heart, liver, skeletal muscle, kidney and pancreas. Detected at low levels in brain, lung and placenta | Kv6.2, KCNF2 | 18q23 | **Q9UJ96** |
| KCNG3 | potassium voltage-gated channel, subfamily G, member 3 | Detected in many parts of the brain with the exception of the cerebellum, in testis, pancreas, lung, kidney, ovary, small intestine, colon, thymus, adrenal gland and spinal cord | Kv6.3 | 2p21 | **Q8TAE7**, Q53SC1 |
| KCNG4 | potassium voltage-gated channel, subfamily G, member 4 | Highly expressed in brain, and at lower levels in liver, small intestine and colon. | Kv6.4 | 16q24.1 | Q8TDN1, Q96H24 |
| KCNH1 | potassium voltage-gated channel, subfamily H (eag-related), member 1 | Highly expressed in brain and in myoblasts at the onset of fusion, but not in other tissues. Detected in HeLa (cervical carcinoma), SH-SY5Y (neuroblastoma ) and MCF-7 (epithelial tumor) cells, but not in normal epithelial cells. | Kv10.1, eag, h-eag, eag1 | 1q32.2 | **O95259** B1AQ26, O76035, Q14CL3 |
| KCNH2 | potassium voltage-gated channel, subfamily H (eag-related), member 2 | Highly expressed in heart and brain. Isoforms USO are frequently overexpresse d in cancer cells | Kv11.1, HERG, erg1 | 7q36.1 | **Q12809** A5H1P7 Q9H3P0 |
| KCNH3 | potassium voltage-gated channel, | Detected only in brain, in particular in the | Kv12.2, BEC1, elk2 | 12q13 | **Q9ULD8** Q9UQ06 |
| | subfamily H (eag-related), member 3 | telencephalon. Detected in the cerebral cortex, occipital pole, frontal and temporal lobe, putamen, amygdala, hippocampus and caudate nucleus | | | |
| KCNH4 | potassium voltage-gated channel, subfamily H (eag-related), member 4 | Detected only in brain, in particular in the telencephalon. Detected in putamen and caudate nucleus, and at lower levels in cerebral cortex, occipital and hippocampus | Kv12.3, elk1 | 17q21 | **Q9UQ05** |
| KCNH5 | potassium voltage-gated channel, subfamily H (eag-related), member 5 | Detected in brain, skeletal muscle, heart, placenta, lung and liver, and at low levels in kidney | Kv10.2, H-EAG2, eag2 | 14q23.1 | **Q8NCM2,** C9JP98 |
| KCNH6 | potassium voltage-gated channel, subfamily H (eag-related), member 6 | Expressed in prolactin-secreting adenomas | Kv11.2, erg2, HERG2 | 17q23.3 | **Q9H252,** Q9BRD7 |
| KCNH7 | potassium voltage-gated channel, subfamily H (eag-related), member 7 | Expressed in prolactin-secreting adenomas | Kv11.3, HERG3, erg3 | 2q24.3 | **Q9NS40,** Q53QU4, Q8IV15 |
| KCNH8 | potassium voltage-gated channel, subfamily H (eag-related), member 8 | Nervous system | Kv12.1, elk3 | 3p24.3 | **Q96L42,** Q59GQ6 |
| KCNQ1 | potassium voltage-gated channel, KQT-like subfamily, member 1 | Abundantly expressed in heart, pancreas, prostate, kidney, small intestine and peripheral blood leukocytes. Less abundant in placenta, lung, spleen, colon, thymus, testis and ovaries. | Kv7.1, KCNA8, KVLQT1, JLNS1, LQT1 | 11 p15.5 | **P51787,** 000347 Q9UMN9 |
| KCNQ2 | potassium voltage-gated channel, KQT-like subfamily, member 2 | In adult and fetal brain. Highly expressed in areas containing neuronal cell bodies, low in spinal chord and corpus callosum. Isoform 2 is preferentially expressed in differentiated neurons. Isoform 6 is prominent in fetal brain, undifferentiated neuroblastoma cells and brain tumors | Kv7.2, ENB1, BFNC, KCNA11, HNSPC | 20q13.33 | **O43526**, O43796, Q99454 |
| KCNQ3 | potassium voltage-gated channel, KQT-like subfamily, member 3 | Brain | Kv7.3 | 8q24 | **O43525**, A2VCT8, B4DJY4, E7EQ89 |
| KCNQ4 | potassium voltage-gated channel, KQT-like subfamily, member 4 | Expressed in the outer, but not the inner, sensory hair cells of the cochlea. Slightly expressed in heart, brain and skeletal muscle. | Kv7.4 | 1p34 | **P56696**, O96025 |
| KCNQ5 | potassium voltage-gated channel, KQT-like subfamily, member 5 | Strongly expressed in brain and skeletal muscle. In brain, expressed in cerebral cortex, occipital pole, frontal lobe and temporal lobe. Lower levels in hippocampus and putamen. Low to undetectable levels in medulla, cerebellum and thalamus. | Kv7.5 | 6q14 | **Q9NR82**, B4DS33. Q9NYA6 |
| KCNS1 | potassium voltage-gated channel, | Detected in all tissues tested with the | Kv9.1 | 20q12 | **A2RUL8,_Q96KK3**, A2RUL9, Q6DJU6, |
| | delayed-rectifier, subfamily S, member 1 | exception of skeletal muscle. Highly expressed in adult and fetal brain, fetal kidney and lung, and adult prostate and testis. | | | **Q92953,** Q7Z7D0, Q9BXD3 |
| KCNS2 | potassium voltage-gated channel, delayed-rectifier, subfamily S, member 2 | | Kv9.2 | 8q22 | **Q9ULS6,** A8KAN1, **Q92953,** Q7Z7D0, Q9BXD3,_**Q14721**, Q14193 |
| KCNS3 | potassium voltage-gated channel, delayed-rectifier, subfamily S, member 3 | Detected in whole normal term placental and placental chorionic plate arteries and veins. Detected in syncytiotrophob last and in blood vessel endothelium within intermediate villi and chorionic plate (at protein level). Detected in most tissues, but not in peripheral blood lymphocytes. The highest levels of expression are in lung | Kv9.3 | 2p24 | **Q9BQ31,** D6W520 Q96B56,_**C9J187**, **L8E8W4,_Q92953,** Q7Z7D0, Q9BXD3 **Q14721**, Q14193 |
| KCNV1 | potassium channel, subfamily V, member 1 | Detected in brain | Kv8.1 | 8q23.2 | **Q6PIU1,** Q9UHJ4, **B2R8Q7,_B4DMC1 Q76FP2** |
| KCNV2 | potassium channel, subfamily V, member 2 | Detected in heart, brain, liver, skeletal muscle, spleen, thymus, prostate, testis, ovary colon, kidney and pancreas | Kv8.2 | 9p24.2 | **Q8TDN2,** Q5T6X0 **P48547,** K4DI87, **Q14721,** Q14193, **Q9H3M0** O43527, Q585L3 |

**Table 9: Potassium channels, inwardly rectifying**

| Approved Symbol | Approved Name | Tissue distribution | Synonyms | Chromosome | Accession numbers on uniprot.org website |
|---|---|---|---|---|---|
| KCNJ1 | potassium inwardly-rectifying channel, subfamily J, member 1 | In the kidney and pancreatic islets. Lower levels in skeletal muscle, pancreas, spleen, brain, heart and liver | Kir1.1, ROMK1 | 11q24 | **P48048,** B2RMR4, Q6LD67, **A0A024R3K6** |
| KCNJ2 | potassium inwardly-rectifying channel, subfamily J, member 2 | Heart, brain, placenta, lung, skeletal muscle, and kidney. Diffusely distributed throughout the brain. | Kir2.1, IRK1, LQT7 | 17q24.3 | **P63252,** 015110, P48049,_**Q9NPI9** |
| KCNJ3 | potassium inwardly-rectifying channel, subfamily J, member 3 | | Kir3.1, GIRK1, KGA | 2q24.1 | **P48549,** B4DEW7, Q8TBI0,_**D2XBF0, D2X9V0** |
| KCNJ4 | potassium inwardly-rectifying channel, subfamily J, member 4 | Heart, skeletal muscle, and several different brain regions including the hippocampus | Kir2.3, HIR, HRK1, hIRK2, IRK3 | 22q13.1 | **P48050,** Q14D44, **A0A024R1L8, P63252,** O15110, P48049 |
| KCNJ5 | potassium inwardly-rectifying channel, subfamily J, member 5 | Islets, exocrine pancreas and heart. Expressed in the adrenal cortex, particularly the zona glomerulosa | Kir3.4, CIR, KATP1, GIRK4, LQT13 | 11q24 | **Q4QRJ2,_P48544,** B2R744 Q92807, **H9A8K9,_H7CGH0, A0A024R3K7** |
| KCNJ6 | potassium inwardly-rectifying channel, subfamily J, member 6 | Widely expressed. Expressed in cells of hematopoietic origin (at protein level). Most abundant in cerebellum, and to a lesser degree in islets and exocrine pancreas | Kir3.2, GIRK2, KATP2, BIR1, hiGIRK2 | 21q22.1 | **P48051** Q3MJ74, Q53WW6, _**B2RA12, Q96L92,** Q32Q36 Q9H3K8 |
| KCNJ8 | potassium inwardly-rectifying channel, subfamily J, member 8 | Predominantly detected in fetal and adult heart | Kir6.1 | 12p12.1 | **Q15842,** 000657, **F5GY12, A0A024RAV6** |
| KCNJ9 | potassium inwardly-rectifying channel, subfamily J, member 9 | Widely expressed. Expressed in cells of hematopoietic origin (at protein level). | Kir3.3, GIRK3 | 1q23.2 | **Q92806,** Q5JW75, **Q96L92,** Q32Q36, Q9H3K8 |
| KCNJ10 | potassium inwardly-rectifying channel, subfamily J, member 10 | | Kir4.1, Kir1.2 | 1q23.2 | **P78508,** A3KME7,Q92808, **Q547K1,_Q9BXC5 Q9NPI9** |
| KCNJ11 | potassium inwardly-rectifying channel, subfamily J, member 11 | | Kir6.2, BIR | 11p15.1 | **Q14654,** B4DWI4,Q8IW96 **E9PPF1,_H0YES9, D2K1F9** |
| KCNJ12 | potassium inwardly-rectifying channel, subfamily J, member 12 | Heart, skeletal muscle, and several different brain regions including the hippocampus | Kir2.2, Kir2.2v, IRK2, hIRK1 | 17p11.1 | **Q14500,** 043401, Q15756, Q8NG63, **B7U540,_ Q9HAP6, P48050,** Q14D44 |
| KCNJ13 | potassium inwardly-rectifying channel, subfamily J, member 13 | Predominantly expressed in small intestine. Expression is also detected in stomach, kidney, and all central nervous system regions tested with the exception of spinal cord | Kir7.1, Kir1.4, LCA16 | 2q37 | **O60928** A0PGH1 Q8N3Y4 **C9JWD6 H7C4D1** |
| KCNJ14 | potassium inwardly-rectifying channel, subfamily J, member 14 | Expressed preferentially in retina | Kir2.4, IRK4 | 19q13 | **Q9UNX9_Q99712** D3DSH5 Q99446 |
| KCNJ15 | potassium inwardly-rectifying channel, subfamily J, member 15 | | Kir4.2, Kir1.3, IRKK | 21q22.2 | **Q99712** D3DSH5 Q99446,_**A8K9U7** |
| KCNJ16 | potassium inwardly-rectifying channel, subfamily J, member 16 | Highly expressed in kidney, pancreas and thyroid gland | Kir5.1, BIR9 | 17q24.3 | **Q9NPI9** |
| KCNJ18 | potassium inwardly-rectifying channel, subfamily J, member 18 | Specifically expressed in skeletal muscle | KIR2.6, TTPP2 | 17p11.2 | **B7U540** |

**Table 10: Potassium channels, two-P**

| Approved Symbol | Approved Name | Tissue distribution | Synonyms | Chromosome | Accession numbers on uniprot.org website |
|---|---|---|---|---|---|
| KCNK1 | potassium channel, subfamily K, member 1 | Widely expressed with high levels in heart and brain and lower levels in placenta, lung, liver and kidney | K2p1.1, DPK, TWIK-1 | 1q42-q43 | **000180**, Q13307, Q5T5E8 |
| KCNK2 | potassium channel, subfamily K, member 2 | | K2p2.1, TREK-1 | 1q41 | **Q6ZW95,_Q13303,** A0AVM9, Q99411 |
| KCNK3 | potassium channel, subfamily K, member 3 | Widespread expression in adult. Strongest expression in pancreas and placenta. Lower expression in brain, lung, prostate, heart, kidney, uterus, small intestine and colon | K2p3.1, TASK, TASK-1 | 2p23 | **O14649,** Q53SU2, **B9EIJ4** |
| KCNK4 | potassium channel, subfamily K, member 4 | | K2p4.1, TRAAK | 11q13 | **Q2YDA1** |
| KCNK5 | potassium channel, subfamily K, member 5 | Abundant expression in kidney, also detected in liver, placenta and small intestine. In the kidney, expression is restricted to the distal tubules and collecting ducts. | K2p5.1, TASK-2 | 6p21 | **O95279** B2RAQ6, B5TJL2, Q5VV76 |
| KCNK6 | potassium channel, subfamily K, member 6 | Widespread expression, detected in all tissues tested except for skeletal muscle. Strongest expression in placenta, pancreas, heart, colon and spleen, lower levels detected in peripheral blood leukocytes, lung, liver, kidney and thymus. Lowest expression detected in brain | K2p6.1, TWIK-2 | 19q13.1 | **Q9Y257** Q9HB47 |
| KCNK7 | potassium channel, subfamily K, member 7 | | K2p7.1 | 11q13 | **Q3MI97** |
| KCNK9 | potassium channel, subfamily K, member 9 | Mainly found in the cerebellum. Also found in adrenal gland, kidney and lung | K2p9.1, TASK3, TASK-3 | 8q24.3 | **Q9NPC2,** Q2M290, Q540F2 |
| KCNK10 | potassium channel, subfamily K, member 10 | Abundantly expressed in pancreas and kidney and to a lower level in brain, testis, colon, and small | K2p10.1, TREK-2, TREK2, PPP1R97 | 14q31 | **P57789,** B2R8T4, Q9HB59 |
| | | intestine. Isoform b is strongly expressed in kidney (primarily in the proximal tubule) and pancreas, whereas isoform c is abundantly expressed in brain | | | |
| KCNK12 | potassium channel, subfamily K, member 12 | | THIK-2, THIK2, K2p12.1 | 2p16.3 | **Q9HB15** |
| KCNK13 | potassium channel, subfamily K, member 13 | | K2p13.1, THIK-1, THIK1 | 14q32.11 | **Q9HB14,** B5TJL8, Q96E79 |
| KCNK15 | potassium channel, subfamily K, member 15 | Detected in pancreas, heart, placenta, lung, liver, kidney, ovary, testis, skeletal muscle and adrenal gland, and at lower levels in prostate, spleen and thyroid gland | K2p15.1, dJ781B1.1, KT3.3, KIAA0237, TASK5, TASK-5 | 20q13.12 | **Q9H427,** Q52LL3, Q9HBC8 |
| KCNK16 | potassium channel, subfamily K, member 16 | Highly expressed in pancreas. | K2p16.1, TALK-1, TALK1 | 6p21.2-p21.1 | **Q96T55**, B5TJL9, Q9H591 |
| KCNK17 | potassium channel, subfamily K, member 17 | | K2p17.1, TALK-2, TALK2, TASK4, TASK-4 | 6p21 | **Q96T54**, E9PB46, **Q9H592,_B2RCT9** |
| KCNK18 | potassium channel, subfamily K, member 18 | Expressed specifically in dorsal root ganglion and trigeminal ganglion neurons. Detected at low levels in spinal cord. | K2p18.1, TRESK-2, TRESK2, TRESK, TRIK | 10q26.11 | **Q7Z418,** Q5SQQ8 |

**Table 11: Hydrogen voltage-gated ion channels**

| Approved Symbol | Approved Name | Tissue distribution | Synonyms | Chromosome | Accession numbers on uniprot.org website |
|---|---|---|---|---|---|
| HVCN1 | hydrogen voltage-gated channel 1 | Enriched in immune tissues, such as lymph nodes, B-lymphocytes, monocytes and spleen | MGC15619, Hv1, VSOP | 12q24.11 | **Q96D96,** A8MQ37, Q96IS5 |

Ligand-dependent ion channels also commonly referred to as ligand-gated ion channels (LICs, LGIC), or as ionotropic receptors are a group of integral membrane proteins which contain a pore which allows the flow of selected ions such as Na⁺, K⁺, Ca²⁺, and/or Cl⁻ to pass through the membrane in response to the binding of a chemical messenger, *i.e.* a ligand.

These channels thus comprise a fourth domain which is extracellular and includes a ligand binding domain. Such ligand may be for example a neurotransmitter. When a presynaptic neuron is excited, it releases a neurotransmitter from vesicles into the synaptic cleft. The neurotransmitter then binds to receptors located on the postsynaptic neuron, causing a conformational change of the ion channels, and leading, upon activation or inhibition, to a flow of ions across the cell membrane. This, in turn, results in either a depolarization, for an excitatory receptor response, or a hyperpolarization, for an inhibitory response. The function of these channels when located within postsynapses is to directly and very quickly convert the chemical signal of presynaptically released neurotransmitter into a postsynaptic electrical signal.

These ligand-gated ion channels are subdivided with respect to the type of ion that they conduct, *e.g*., anionic or cationic and further into families defined by the endogenous ligand. They comprise cation-selective receptors including acetylcholine receptors, 5-HT3 receptors, ionotropic glutamate receptors as well as P2X receptors and anion-selective receptors including GABAA receptors, glycine receptors and zinc-activated channels. In terms of configuration, nicotinic acetylcholine receptors, 5-HT3 receptors, GABAA (gamma-aminobutyric acid A) receptors, glycine receptors, and the additional zinc-activated channel are pentameric structures and are frequently referred to as the Cys-loop receptors due to the presence of a defining loop of residues formed by a disulphide bond between two cysteine residues in the N terminal extracellular domains of their constituent subunits. The ionotropic glutamate receptors and P2X receptors are tetrameric and trimeric structures, respectively. A binding site in the extracellular N-terminal ligand-binding domain gives them receptor specificity for specific endogenous ligands including acetylcholine (AcCh), serotonin, glycine, glutamate and γ-aminobutyric acid (GABA), etc...

5-HT₃ receptors or 5-hydroxytryptamine (serotonin) receptors exist as a pentamer of 4TM subunits that form an intrinsic cation selective. Five human 5-HT₃ receptor subunits including 5-HT_{3A} (https://www.uniprot.org/uniprot/P46098), 5-HT_{3B} (https://www.uniprot.org/uniprot/P46098), 5-HT_{3C} (https://www.uniprot.org/uniprot/Q8WXA8), 5-HT_{3D} (https://www.uniprot.org/uniprot/Q70Z44), 5-HT_{3E} (https://www.uniprot.org/uniprot/A5X5Y0) have been cloned and homo-oligomeric assemblies of 5-HT₃A and hetero-oligomeric assemblies of 5-HT₃A and 5-HT₃B subunits have been fully characterized (5-HT₃A and 5-HT₃AB receptors). The 5-HT₃C subunit, as well as 5-HT₃D and 5-HT₃E subunits, like the 5-HT₃B subunit, do not form functional homomers, but assemble with the 5-HT₃A subunit to influence its functional expression rather than pharmacological profile. The serotonin receptors are a group of G protein-coupled receptor and ligand-gated ion channels found in the central and peripheral nervous systems which mediate both excitatory and inhibitory neurotransmission. They are activated by the neurotransmitter serotonin, which acts as their natural ligand, and they modulate the release of many neurotransmitters including glutamate, GABA, dopamine, epinephrine/norepinephrine, and acetylcholine as well as many hormones, such as oxytocin, prolactin, vasopressin, cortisol corticotropin, and substance P. These receptors influence various biological and neurological processes such as aggression, anxiety, appetite, cognition, learning, memory, mood, nausea, and sleep.

Zinc-activated ion channel (ZAC) is also known as ZAC1, L2m LICZ, LICZ1 (https://www.uniprot.org/uniprot/Q401N2). ZAC forms a cation-permeable ligand-gated ion channel of the cys-loop family. This channel most likely exists as a homopentamer of four transmembrane subunits that form an intrinsic cation selective channel equipermeable to Na⁺, K⁺ and Cs⁺, but impermeable to Ca²⁺ and Mg²⁺. ZAC are expressed in prostate, thyroid, trachea, lung, brain (adult and fetal), spinal cord, skeletal muscle, heart, placenta, pancreas, liver, kidney and stomach. The endogenous ligand for ZAC is thought to be Zn²⁺, although ZAC has also been found to activate spontaneously.

GABA_{A} (gamma-aminobutyric acid A) receptors or channels are ionotropic receptors. They exist as pentamers of four transmembrane subunits including six alpha subunits: α1 to α6 subunits (https://www.uniprot.org/uniprot/P14867, P47869, P34903, P48169, P31644, Q16445), three beta subunits: β1 to β3 subunits (https://www.uniprot.org/uniprot/P14867, P47870, P28472), three gamma subunits: γ1 to γ3 subunits (https://www.uniprot.org/uniprot/Q8N1C3, P18507, Q99928), one delta (δ) subunit (https://www.uniprot.org/uniprot/O14764), one epsilon (ε) subunit (https://www.uniprot.org/uniprot/P78334), one pi (π) subunit (https://www.uniprot.org/uniprot/O00591), one theta (θ) subunit (https://www.uniprot.org/uniprot/Q9UN88), and three rho subunits: ρ1 to p3) receptor subunits (https://www.uniprot.org/uniprot/P24046, P28476, A8MPY1). Many GABA_{A} receptor subtypes contain α-, β- and γ-subunits with the stoichiometry 2α.2β.1γ. In the CNS however, α1β2γ2 heterooligomer constitutes the largest population of GABA_{A} receptors in the CNS, followed by the α2β3γ2 and α3β3γ2 isoforms. Currently, eleven native GABA_{A} receptors are classed as conclusively identified, *i.e*., α1β2γ2, α1βγ2, α3βγ2, α4βγ2, α4β2δ, α4β3δ, α5βγ2, α6βγ2, α6β2δ, α6β3δ and ρ. The endogenous ligand is γ-aminobutyric acid (GABA) which is the major inhibitory neurotransmitter in the central nervous system. Upon activation, the GABA_{A} receptor selectively conducts chloride (Cl⁻) through its pore. Several inhibitory drugs have been identified including muscimol, gaboxadol, bicuculline, the benzodiazepines, nonbenzodiazepines, alprazolam, diazepam, and picrotoxin.

The glycine receptor also referred to as GlyR or GLRi is the receptor of the amino acid neurotransmitter glycine. GlyR is an ionotropic receptor that produces its effects through chloride current. The receptor is expressed either as a homo-pentamer of α subunits, or a complex of 2α and 3β subunits. Amino acid sequences of glycine receptor subunits α1 to α3 and β are provided in https://www.uniprot.org/uniprot/P23415, P23416, 075311, and P48167 respectively. It is one of the most widely distributed inhibitory receptors in the central nervous system and has important roles in a variety of physiological processes, especially in mediating inhibitory neurotransmission in the spinal cord and brainstem. The receptor can be activated by a range of simple amino acids including glycine, β-alanine and taurine, and can be selectively blocked by the high-affinity competitive antagonist strychnine. Caffeine is a competitive antagonist of GlyR.

The ionotropic glutamate receptors bind the neurotransmitter glutamate. They form tetramers with each subunit consisting of an extracellular amino terminal domain (ATD), which is involved in subunit assembly, an extracellular ligand binding domain (LBD) which binds glutamate, and a transmembrane domain (TMD) which forms the ion channel. Each subunit of the tetramer has a binding site for glutamate formed by the two LBD sections forming a clamshell like shape. Only two of these sites in the tetramer need to be occupied to open the ion channel. Ionotropic glutamate receptors comprise members of the NMDA (N-methyl-D-aspartate), AMPA (α-amino-3-hydroxy-5-methyl-4-isoxazoleproprionic acid) and kainate receptor classes.

NMDA receptors assemble as heteromers comprising from GluN1 (https://www.uniprot.org/uniprot/Q05586), GluN2A (https://www.uniprot.org/uniprot/Q12879), GluN2B (https://www.uniprot.org/uniprot/Q13224), GluN2C, GluN2D (https://www.uniprot.org/uniprot/Q12879), GluN3A (https://www.uniprot.org/uniprot/Q8TCU5) and GluN3B (https://www.uniprot.org/uniprot/O60391) subunits. AMPA receptors assemble as homomers, or heteromers comprising GluA1, GluA2, GluA3 and GluA4 subunits. kainate receptors can be expressed as homomers of GluK1 subunit (https://www.uniprot.org/uniprot/P39086), GluK2 subunit (https://www.uniprot.org/uniprot/ Q13002) or GluK3 subunit (https://www.uniprot.org/uniprot/Q13003). GluK1-3 subunits are also capable of assembling into heterotetramers, *e.g.*, GluK1/K2.

P2X receptors are ATP-gated channels which open in response to binding the extracellular nucleotide ATP. They form trimers with two transmembrane domains with C and N termini located on the intracellular side, gating Na⁺, K⁺ and Ca²⁺. Native P2X receptors may occur as either homotrimers or heterotrimers including from P2X1 to 7 subunits (https://www.uniprot.org/uniprot/P51575, Q9UBL9, P56373, Q99571, Q93086, 015547, Q99572).

Nicotinic acetylcholine receptors (nAChR) are members of the Cys-loop family, consisting of a pentamer of protein subunits (typically ααβγδ). Subunits may be of class alpha (α1 to α10) referred to as ACHRA (https://www.uniprot.org/uniprot/P02708), class beta (β1 to β4) referred to as ACHRB (https://www.uniprot.org/uniprot/P11230), class gamma(γ) or ACHRG (https://www.uniprot.org/uniprot/P07510), class delta(δ) or ACHRD (https://www.uniprot.org/uniprot/Q07001), class epsilon (ε) or ACHRE (https://www.uniprot.org/uniprot/Q04844). Binding sites for acetylcholine are on the interface of each alpha subunit. When the acetylcholine binds, it alters the receptor's configuration and causes the constriction in the pore of approximately 3 angstroms to widen to approximately 8 angstroms so that ions can pass through. This pore allows Na⁺ ions to flow down their electrochemical gradient into the cell. With a sufficient number of channels opening at once, the inward flow of positive charges carried by Na⁺ ions depolarizes the postsynaptic membrane sufficiently to initiate an action potential. nAChRs respond to the neurotransmitter acetylcholine and to drugs such as the agonist nicotine. They are found in the central and peripheral nervous system, muscle, and many other tissues of many organisms. At the neuromuscular junction they are the primary receptor in muscle for motor nerve-muscle communication that controls muscle contraction. In the peripheral nervous system: they transmit outgoing signals from the presynaptic to the postsynaptic cells within the sympathetic and parasympathetic nervous system, and they are the receptors found on skeletal muscle that receive acetylcholine released to signal for muscular contraction.

Therefore, according to the present invention, the ion conducting channel fusion subunits are thus subunits of the ion conducting channels, either ligand-dependent ion channels or voltage-dependent ion channel as described above, which is bound or coupled in C-terminal or N-terminal to the probe comprising an ion sensor which is sandwiched between at least one bioluminescent donor molecule and at least one fluorescent acceptor molecule. Alternatively, the ion sensor sandwiched by the bioluminescent donor and the fluorescent acceptor molecule may also be inserted inside an intracellular or an extracellular loop of an ion conducting channel subunit. When the subunits of said ion conducting channels are expressed in cells, they may thus reassemble to a form an active ion conducting channel fusion protein which may be used for HTS method according to the present invention. Such channel fusion protein may be formed by the same types of subunits and is a homopolymer channel (homodimer, homotetramer, etc..) or by different types of subunits and is a heteropolymer channel (heterodimer, heterotetramer, etc...).

Bioluminescent donor molecule and fluorescent acceptor molecule are selected so that the emission spectrum of the bioluminescent donor molecule overlaps with the absorbance spectrum of the acceptor molecule, so the light energy delivered by the bioluminescent donor molecule is at a wavelength that is able to excite the acceptor molecule, and thus making it possible the non-radiative energy transfer between the bioluminescent energy donor and the fluorescent acceptor through nonradiative dipole-dipole coupling.

Bioluminescent donor refers to any moiety capable of acting on a suitable substrate to transform chemical energy into light energy. For example, it may refer to an enzyme which converts a substrate into an activated product which then releases energy as it relaxes. The activated product (generated by the activity of the bioluminescent protein on the substrate) is the source of the bioluminescent protein-generated luminescence that is transferred to the acceptor molecule.

Several bioluminescent donor molecules may be used in the present invention. Light-emitting systems have been known and isolated from many luminescent organisms including bacteria, protozoa, coelenterates, molluscs, fish, millipedes, flies, fungi, worms, crustaceans, and beetles, particularly click beetles of genus Pyrophorus and the fireflies of the genera Photinus, Photuris, and Luciola. Additional organisms displaying bioluminescence are listed in international publications WO 00/024878 and WO 99/049019.

One well characterized example is the class of proteins known as luciferases. Luciferase proteins catalyze an energy-yielding chemical reaction in which a specific biochemical substance, a luciferin (a naturally occurring substrate), is oxidized by an enzyme having a luciferase activity. Both prokaryotic and eukaryotic organisms including species of bacteria, algae, fungi, insects, fish and other marine forms can emit light energy in this manner and each has specific luciferase activities and luciferins, which are chemically distinct from those of other organisms. Luciferin/luciferase systems are very diverse in form, chemistry and function. For example, there are luciferase activities which facilitate continuous chemiluminescence, as exhibited by some bacteria and mushrooms, and those which are adapted to facilitate sporadic, or stimuli induced, emissions, as in the case of dinoflagellate algae. As a phenomenon, which entails the transformation of chemical energy into light energy, bioluminescence is not restricted to living organisms, nor does it require the presence of living organisms. It is simply a type of chemiluminescent reaction that requires a luciferase activity, which at one stage or another had its origins from a biological catalyst. Hence the preservation or construction of the essential activities and chemicals suffices to have the means to give rise to bioluminescent phenomena.

Such bioluminescent donor molecules may be for example luciferases which are well-known in the art. Luciferases are enzymes or oxygenases that catalyze a light emitting reaction, *e.g*., a bioluminescent reaction.

All known kinds of luciferases, either naturally occurring luciferases or mutated luciferases are encompassed within the scope of the invention.

Examples of bioluminescent proteins with luciferase activity may be found in US 5,229,285, 5,219,737, 5,843,746, 5,196,524, and 5,670,356. Two of the most widely used luciferases are: (i) Renilla luciferase (from R. reniformis), a 35 kDa protein, which uses coelenterazine as a substrate and emits light at 480 nm; and (ii) Firefly luciferase (from Photinus pyralis), a 61 kDa protein, which uses luciferin as a substrate and emits light at 560 nm.

Bioluminescent donor molecule may be a luciferase chosen among Renilla luciferase, Firefly luciferase, Coelenterate luciferase, North American glow worm luciferase, click beetle luciferase, railroad worm luciferase, Gaussia luciferase, Aequorin, Arachnocampa luciferase, luciferase derived from the copepods like Gaussia princeps, Pleuromamma abdominalis, Metridia pacifica, Metridia curticauda, Metridia asymmetrica, Metridia okhotensis, Metridia longa, Lucicutia ovaliformis, Heterorhabdus tanneri, Pleuromamma scutullata, or a biologically active variant or fragment thereof.

A preferred selection of molecular weight range of luciferases for use according to the present invention may be in the range of 15 to 70 kDa, or of 15 to 60 kDa, or of 15 to 50kDa, or 15 to 40kDa, or 15-30kDa.

Among preferred luciferases, we may cite GLuc, NanoLuc (NLuc), MLuc7, HtLuc, LoLuc, PaLuc1, PaLuc2, MpLuc1, McLuc1, MaLuc1, MoLuc1, MoLuc2, MLuc39, PsLuc1, LocLuc1-3, HtLuc2 Renilla, TurboLuc16 (TLuc) or homologs or orthologs thereof or mutants or functional derivatives thereof. In various embodiments, mutants or functional derivatives of the luciferases retain at least 100%, 90%, 80%, 70%, 60% or 50% of the bioluminescence activity from which the mutant or function derivative is derived. As described in Takenaka et al. (Mol Biol Evol 29(6): 1669-1681, 2012), the copepod luciferases comprise two domains and each domain includes conserved sequences across the various luciferases. In some embodiments, any luciferase comprising the consensus sequence C-x(3)-C-L-x(2)-L-x(4)-C-x(8)-P-x-R-C(SEQ ID NO: 2437) in each of domains 1 and 2 as described by Takenaka et al., may be used in the methods described herein.

Most preferred luciferases may include nanoluciferase which is a protein derived from the 19 kDa subunit of a luciferase extracted from the deep-sea shrimp *Oplophorus gracilirostris*, the second subunit being a 35 kDa protein as described by S. Inouye et al. (FEBS Letters, 481 (2000) 19-25). This nanoluciferase is marketed under the commercial name NanoLuc® by the company Promega, and it utilizes novel substrates (furimazine or bisdeoxycoelenterazine as well as 6h-f-coelenterazine) to produce high intensity, glow-type luminescence as described by Hall M. et al. (ACS Chem. Biol. 2012, 7, 1848-1857) and in international publication WO2012/061530.

Alternative, non-luciferase, bioluminescent molecules may be any enzymes which can act on suitable substrates to generate a luminescent signal. Specific examples of such enzymes are β-galactosidase, lactamase, horseradish peroxidase, alkaline phosphatase, β-glucuronidase, or β-glucosidase. Synthetic luminescent substrates for these enzymes are well known in the art and are commercially available from companies, such as Tropix Inc. (Bedford, MA, USA).

By way of examples, several bioluminescent donor molecules have been listed in the following Table 12.

**Table 12: Examples of bioluminescent molecules**

| **Species** | **Name** | **Organism** | **MW kDa×10⁻³** | **Emission (nm)** | **Substrate** |
|---|---|---|---|---|---|
| nsect | FFluc | *Photinus pyralis* (North American Firefly) | ∼61 | 560 | D-(-)-2-(6'-hydroxybenzo-thiazolyl)-Δ²- thiazoline-4-carboxylic acid, HBTTCA, (C₁₁ H₈N₂O₃S₂)(Luciferin) |
| nsect | FF'luc | *Luciola cruciate* (Japanese Firefly) | | 560-590 (many mutants) | Luciferin |
| nsect | | Phengodid beetles(railroad worms) | | | |
| nsect | | *Arachnocampa* sp. | | | Luciferin |
| nsect | | *Orphelia fultoni* (North American glow worm) | | | |
| nsect | Clluc | *Pyrophorus plagiophthalamus* | | 546, 560 578 and 593 | Luciferin |
| Jelly Fish | Aequorea | *Aequorea* | 44.9 | 460-470 | Coelenterazine |
| Sea Pansy | Rluc | *Renilla Reniformis* | 36 | 480 | Coelenterazine |
| Sea Pansy (modified) | Rluc8 | *Renilla Reniformis(modified)* | 36 | 487(peak) | Coelenterazine/Deep Blue C |
| Sea Pansy | Rmluc | *Renilla mullerei* | 36.1 | ∼480 | Coelenterazine |
| Sea Pansy | | *Renilla kollikeri* | | | |
| Crustacea (shrimp) | Vluc | *Vargula hilgendorfii* | ∼62 | ∼460 | coelenterazine |
| Crustacea | | *Cypridina(sea firefly)* | 75 | 460 | coelenterazine |
| Dinofagellate (marine algae) | | *Gonyaulax polyedra* | 130 | ∼475 | Tetrapyrrole |
| Mollusc | | *Latia (Freshwater limpet)* | 170 | 500 | Enol formate, terpene, aldehyde |
| Hydroid | | Obelia *biscuspidata* | ∼20 | ∼470 | Coelenterazine |
| Shrimp | | *Oplophorus gracilorostris* | 31 | 462 | Coelenterazine |
| Others | Ptluc | *Ptilosarcus* | | ∼490 | Coelenterazine |
| Others | Glue | *Gaussia* | ∼20 | ∼475 | Coelenterazine |
| | Plluc | *Pleuromamma* | 22.6 | ∼475 | Coelenterazine |
| | | | | | |

The choice of the substrate of the bioluminescent donor molecule can impact on the wavelength and the intensity of the light generated by the bioluminescent protein. A widely known substrate is coelenterazine which occurs in cnidarians, copepods, chaetognaths, ctenophores, decapod shrimps, mysid shrimps, radiolarians and some fish taxa. For *Renilla luciferase* for example, coelenterazine analogues/derivatives are available that result in light emission between 418 and 512 nm. A coelenterazine analogue/derivative (400A, DeepBlueC) has been described emitting light at 400 nm with *Renilla luciferase* (WO01/46691). Other examples of coelenterazine analogues/derivatives are EnduRen and ViviRen.

Luciferins are light-emitting biological pigments found in organisms capable of bioluminescence, which are oxidized in the presence of the enzyme luciferase to produce oxyluciferin and energy in the form of light. Luciferin, or 2-(6-hydroxybenzothiazol-2-yl)-2-thiazoline-4-carboxylic acid, was first isolated from the firefly *Photinus pyralis.* Since then, various forms of luciferin have been discovered and studied from various organisms, mainly from the ocean, for example fish and squid, however, many have been identified for example in worms, beetles and various other insects.

There are at least five general types of luciferin, which are each chemically different and catalyzed by chemically and structurally different luciferases that employ a wide range of different cofactors. First, is firefly luciferin, the substrate of firefly luciferase, which requires ATP for catalysis (EC 1.13.12.7). Second, is bacterial luciferin, also found in some squid and fish, that consists of a long chain aldehyde and a reduced riboflavin phosphate. Bacterial luciferase is FMNH-dependent. Third, is dinoflagellate luciferin, a tetrapyrrolic chlorophyll derivative found in dinoflagellates (marine plankton), the organisms responsible for night-time ocean phosphorescence. Dinoflagellate luciferase catalyzes the oxidation of dinoflagellate luciferin and consists of three identical and catalytically active domains. Fourth, is the imidazolopyrazine vargulin, which is found in certain ostracods and deep-sea fish, for example, Porichthys. Finally, coelanterazine (an imidazolpyrazine), the light-emitter of the protein aequorin, found in radiolarians, ctenophores, cnidarians, squid, copepods, chaetognaths, fish and shrimp.

Several acceptor molecules either protein or non-proteinaceous may be used in the intramolecular novel BRET probe according to the present invention.

Acceptor proteins may include for example green fluorescent protein (GFP), variant of green fluorescent protein (such as GFP10), blue fluorescent protein (BFP), cyan fluorescent protein (CFP), yellow fluorescent protein (YFP), enhanced GFP (EGFP), enhanced CFP (ECFP), enhanced YFP (EYFP), GFPS65T, mAmetrine, LSS-mOrange, LSS-mKate, Emerald, Topaz, GFPuv, destabilised EGFP (dEGFP), destabilised ECFP (dECFP), destabilised EYFP (dEYFP), HcRed, t-HcRed, DsRed, DsRed2, mRFPI, pocilloporin, Renilla GFP, Monster GFP, paGFP, Kaede protein, a Phycobiliprotein, TagCFP, mTagCFP2, Czurite, ECFP2, mKalamal, Sirius, Sapphire, T-Sapphire, ECFP, Cerulean, SCFP3C, mTurquoise, mTurquoise2, monomeric Midoriishi-Cyan, TagCFP, mTFPI, EGFP, Emerald, Superfolder GFP, Monomeric Czami Green, TagGFP2, mUKG, mWasabi, Clover, Citrine, Venus, SYFP2, TagYFP, Monomeric Kusabira-Orange, 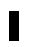 ηKOκ, mK02, mOrange, mOrange2, mRaspberry, mCherry, mStrawberry, mScarlet, mTangerine, tdTomato, TagRFP, TagRFP-T, mCpple, mRuby, mRuby2, mPlum, HcRed-Tandem, mKate2, mNeptune, NirFP, TagRFP657, IFP1.4 and iRFP or a biologically active variant or fragment of any one thereof.

The most frequently used bioluminescent or fluorophore is the green fluorescent protein from the jellyfish Aequorea victoria and numerous other variants (GFPs) obtained for example by mutagenesis and chimeric protein technologies. GFPs are classified based on the distinctive component of their chromophores, each class having distinct excitation and emission wavelengths: class 1, wild-type mixture of neutral phenol and anionic phenolate: class 2, phenolate anion: class 3, neutral phenol: class 4, phenolate anion with stacked s-electron system: class 5, indole: class 6, imidazole: and class 7, phenyl.

Preferred fluorescent molecules or fluorescent acceptor molecules may be the mNeonGreen, which is a monomeric yellow-green fluorescent protein derived from a tetrameric fluorescent protein from the cephalochordate *Branchiostoma lanceolatum* and is described as the "brightest monomeric green or yellow fluorescent protein." The previously developed mNeonGreen monomeric protein is called here the wild-type mNeonGreen. Also included herein are mNeonGreen variants that are improved compared to the wild-type protein in their photo stability and fluorescence. See for example mNeonGreen variants of a wild-type mNeonGreen of SEQ ID NO: 65 as reported in international publication WO2019/055498, which comprise one or more mutations selected from V52H, D53E, N65Y, E69M or E69Q, A110Q, S 1311 or S 131C, T139R, S 143D, K152H, A158P, T164Q, S 166K, A169G, W172P, S 175E, K177M, T178L, T188D or T188E, K190T, T194K, G196K or G196E, N197D, S203C, T204Q, T208H, N218D, and Y226F.

Other representative acceptor molecules may include, but are not limited to sgGFP, sgBFP, BFP blue shifted GFP (Y66H), Cyan GFP, DsRed, monomeric RFP, EBFP, ECFP, GFP (S65T), GFP red shifted (rsGFP), non-UV excitation (wtGFP), UV excitation (wtGFP), GFPuv, HcRed, rsGFP, Sapphire GFP, sgBFP™, sgBFP™ (super glow BFP), sgGFP™, sgGFP™ (super glow GFP), Yellow GFP, semiconductor nanoparticles (*e.g*., raman nanoparticles), 1,5 IAEDANS; 1,8-ANS; 4-Methylumbelliferone; 5-carboxy-2,7-dichlorofluorescein; 5-Carboxyfluorescein (5-FAM); 5-Carboxynapthofluorescein; 5-Carboxytetramethylrhodamine (5-TAMRA); 5-FAM (5-Carboxyfluorescein); 5-HAT (Hydroxy Tryptamine); 5-Hydroxy Tryptamine (HAT); 5-ROX (carboxy-X-rhodamine); 5-TAMRA (5-Carboxytetramethylrhodamine); 6-Carboxyrhodamine 6G; 6-CR 6G; 6-JOE; 7-Amino-4-methylcoumarin; 7-Aminoactinomycin D (7-AAD); 7-Hydroxy-4-methylcoumarin; 9-Amino-6-chloro-2-methoxyacridine; ABQ; Acid Fuchsin; ACMA (9-Amino-6-chloro-2-methoxyacridine); Acridine Orange; Acridine Red; Acridine Yellow; Acriflavin; Acriflavin Feulgen SITSA; Aequorin (Photoprotein); AFPs (AutoFluorescent Protein; Quantum Biotechnologies); Alexa Fluor 350™; Alexa Fluor 430™; Alexa Fluor 488™; Alexa Fluor 532™; Alexa Fluor 546™; Alexa Fluor 568™; Alexa Fluor 594™; Alexa Fluor 633™; Alexa Fluor 647™; Alexa Fluor 660™; Alexa Fluor 680™; Alizarin Complexon; Alizarin Red; Allophycocyanin (APC); AMC, AMCA-S; AMCA (Aminomethylcoumarin); AMCA-X; Aminoactinomycin D; Aminocoumarin; Aminomethylcoumarin (AMCA); Anilin Blue; Anthrocyl stearate; APC (Allophycocyanin); APC-Cy7; APTRA-BTC; APTS; Astrazon Brilliant Red 4G; Astrazon Orange R; Astrazon Red 6B; Astrazon Yellow 7 GLL; Atabrine; ATTO-TAG™ CBQCA; ATTO-TAG™ FQ; Auramine; Aurophosphine G; Aurophosphine; BAO 9 (Bisaminophenyloxadiazole); BCECF (high pH); BCECF (low pH); Berberine Sulphate; Beta Lactamase; Bimane; Bisbenzamide; Bisbenzimide (Hoechst); bis-BTC; Blancophor FFG; BlancophorSV; BOBO™-1; BOBO™-3; Bodipy 492/515; Bodipy 493/503; Bodipy 500/510; Bodipy 505/515; Bodipy 530/550; Bodipy 542/563; Bodipy 558/568; Bodipy 564/570; Bodipy 576/589; Bodipy 581/591; Bodipy 630/650-X; Bodipy 650/665-X; Bodipy 665/676; Bodipy FI; Bodipy FLATP; Bodipy FI-Ceramide; Bodipy R6G SE; Bodipy TMR; Bodipy TMR-X conjugate; Bodipy TMR-X, SE; Bodipy TR; Bodipy TR ATP; Bodipy TR-X SE; BO-PRO™-1; BO-PRO™-3; Brilliant Sulphoflavin FF; BTC; BTC-5N; Calcein; Calcein Blue; Calcium Crimson™; Calcium Green; Calcium Green-1 Ca²⁺Dye; Calcium Green-2 Ca²⁺; Calcium Green-5N Ca²⁺; Calcium Green-C18 Ca²⁺; Calcium Orange; Calcofluor White; Carboxy-X-rhodamine (5-ROX); Cascade Blue™; Cascade Yellow; Catecholamine; CCF2 (GeneBlazer); CFDA; Chlorophyll; Chromomycin A; Chromomycin A; CL-NERF; CMFDA; Coumarin Phalloidin; C-phycocyanine; CPM Methylcoumarin; CTC; CTC Formazan; Cy2™; Cy3.18; Cy3.5™; Cy3™; Cy5.18; Cy5.5™; Cy5™; Cy7™; cyclic AMP Fluorosensor (FiCRhR); Dabcyl; Dansyl; Dansyl Amine; Dansyl Cadaverine; Dansyl Chloride; Dansyl DHPE; Dansyl fluoride; DAPI; Dapoxyl; Dapoxyl 2; Dapoxyl 3' DCFDA; DCFH (Dichlorodihydrofluorescein Diacetate); DDAO; DHR (Dihydorhodamine 123); Di-4-ANEPPS; Di-8-ANEPPS (non-ratio); DiA (4-Di-16-ASP); Dichlorodihydrofluorescein Diacetate (DCFH); DiD-Lipophilic Tracer; DiD (DilC18(5)); DIDS; Dihydorhodamine 123 (DHR); Dil (DilC18(3)); Dinitrophenol; DiO (DiOC18(3)); DiR; DiR (DilC18(7)); DM-NERF (high pH); DNP; Dopamine; DTAF; DY-630-NHS; DY-635-NHS; ELF 97; Eosin; Erythrosin; Erythrosin ITC; Ethidium Bromide; Ethidium homodimer-1 (EthD-1); Euchrysin; EukoLight; Europium (III) chloride; EYFP; Fast Blue; FDA; Feulgen (Pararosaniline); FIF (Formaldehyd Induced Fluorescence); FITC; Flazo Orange; Fluo-3; Fluo-4; Fluorescein (FITC); Fluorescein Diacetate; Fluoro-Emerald; Fluoro-Gold (Hydroxystilbamidine); Fluor-Ruby; FluorX; FM 1-43™; FM 4-46; Fura Red™ (high pH); Fura Red™/Fluo-3; Fura-2; Fura-2/BCECF; Genacryl Brilliant Red B; Genacryl Brilliant Yellow 10GF; Genacryl Pink 3G; Genacryl Yellow 5GF; GeneBlazer (CCF2); Gloxalic Acid; Granular blue; Haematoporphyrin; Hoechst 33258; Hoechst 33342; Hoechst 34580; HPTS; Hydroxycoumarin; Hydroxystilbamidine (FluoroGold); Hydroxytryptamine; Indo-1, high calcium; Indo-1, low calcium; Indodicarbocyanine (DiD); Indotricarbocyanine (DiR); Intrawhite Cf; JC-1; JO-JO-1; JO-PRO-1; LaserPro; Laurodan; LDS 751 (DNA); LDS 751 (RNA); Leucophor PAF; Leucophor SF; Leucophor WS; Lissamine Rhodamine; Lissamine Rhodamine B; Calcein/Ethidium homodimer; LOLO-1; LO-PRO-1; Lucifer Yellow; Lyso Tracker Blue; Lyso Tracker Blue-White; Lyso Tracker Green; Lyso Tracker Red; Lyso Tracker Yellow; LysoSensor Blue; LysoSensor Green; LysoSensorYellow/Blue; Mag Green; Magdala Red (Phloxin B); Mag-Fura Red; Mag-Fura-2; Mag-Fura-5; Mag-Indo-1; Magnesium Green; Magnesium Orange; Malachite Green; Marina Blue; Maxilon Brilliant Flavin 10 GFF; Maxilon Brilliant Flavin 8 GFF; Merocyanin; Methoxycoumarin; Mitotracker Green FM; Mitotracker Orange; Mitotracker Red; Mitramycin; Monobromobimane; Monobromobimane (mBBr-GSH); Monochlorobimane; MPS (Methyl Green Pyronine Stilbene); NBD; NBD Amine; Nile Red; Nitrobenzoxadidole; Noradrenaline; Nuclear Fast Red; Nuclear Yellow; Nylosan Brilliant lavin E8G; Oregon Green; Oregon Green 488-X; Oregon Green™; Oregon Green™ 488; Oregon Green™ 500; Oregon Green™ 514; Pacific Blue; Pararosaniline (Feulgen); PBFI; PE-Cy5; PE-Cy7; PerCP; PerCP-Cy5.5; PE-TexasRed [Red 613]; Phloxin B (Magdala Red); Phorwite AR; Phorwite BKL; Phorwite Rev; Phorwite RPA; Phosphine 3R; PhotoResist; Phycoerythrin B [PE]; Phycoerythrin R [PE]; PKH26 (Sigma); PKH67; PMIA; Pontochrome Blue Black; POPO-1; POPO-3; PO-PRO-1; PO-PRO-3; Primuline; Procion Yellow; Propidium lodid (PI); PyMPO; Pyrene; Pyronine; Pyronine B; Pyrozal Brilliant Flavin 7GF; QSY 7; Quinacrine Mustard; Red 613 [PE-TexasRed]; Resorufin; RH 414; Rhod-2; Rhodamine; Rhodamine 110; Rhodamine 123; Rhodamine 5 GLD; Rhodamine 6G; Rhodamine B; Rhodamine B 200; Rhodamine B extra; Rhodamine BB; Rhodamine BG; Rhodamine Green; Rhodamine Phallicidine; Rhodamine Phalloidine; Rhodamine Red; Rhodamine WT; Rose Bengal; R-phycocyanine; R-phycoerythrin (PE); S65A; S65C; S65L; S65T; SBFI; Serotonin; Sevron Brilliant Red 2B; Sevron Brilliant Red 4G; Sevron Brilliant Red B; Sevron Orange; Sevron Yellow L; SITS; SITS (Primuline); SITS (Stilbene Isothiosulphonic Acid); SNAFL calcein; SNAFL-1; SNAFL-2; SNARF calcein; SNARF1; Sodium Green; SpectrumAqua; SpectrumGreen; SpectrumOrange; Spectrum Red; SPQ (6-methoxy-N-(3-sulfopropyl)quinolinium); Stilbene; Sulphorhodamine B can C; Sulphorhodamine Extra; SYTO 11; SYTO 12; SYTO 13; SYTO 14; SYTO 15; SYTO 16; SYTO 17; SYTO 18; SYTO 20; SYTO 21; SYTO 22; SYTO 23; SYTO 24; SYTO 25; SYTO 40; SYTO 41; SYTO 42; SYTO 43; SYTO 44; SYTO 45; SYTO 59; SYTO 60; SYTO 61; SYTO 62; SYTO 63; SYTO 64; SYTO 80; SYTO 81; SYTO 82; SYTO 83; SYTO 84; SYTO 85; SYTOX Blue; SYTOX Green; SYTOX Orange; Tetracycline; Tetramethylrhodamine (TRITC); Texas Red™; Texas Red-X™ conjugate; Thiadicarbocyanine (DiSC3); Thiazine Red R; Thiazole Orange; Thioflavin 5; Thioflavin S; Thioflavin TCN; Thiolyte; Thiozole Orange; Tinopol CBS (Calcofluor White); TMR; TO-PRO-1; TO-PRO-3; TO-PRO-5; TOTO-1; TOTO-3; TriColor (PE-Cy5); TRITC TetramethylRodaminelsoThioCyanate; True Blue; TruRed; Ultralite; Uranine B; Uvitex SFC; WW 781; X-Rhodamine; XRITC; Xylene Orange; Y66F; Y66H; Y66W; YO-PRO-1; YO-PRO-3; YOYO-1 ; YOYO-3 ,Sybr Green, Thiazole orange (interchelating dyes), or combinations thereof.

Alternatively, the acceptor molecule may be a fluorescent nanocrystal. Nanocrystals have several advantages over organic molecules as fluorescent labels, including resistance to photodegradation, improved brightness, non- toxicity, and size dependent, narrow emission spectra that enables the monitoring of several processes simultaneously. Additionally, the absorption spectrum of nanocrystals is continuous above the first peak, enabling all sizes, and hence all colors, to be excited with a single excitation wavelength.

The acceptor molecule may also be a fluorescent microsphere. These are typically made from polymers and contain fluorescent molecules (for example fluorescein GFP or YFP) incorporated into the polymer matrix, which can be conjugated to a variety of reagents. Fluorescent microspheres may be labelled internally or on the surface. Internal labelling produces very bright and stable particles with typically narrow fluorescent emission spectra. With internal labelling, surface groups remain available for conjugating ligands (for example, proteins) to the surface of the bead. Internally labelled beads are used extensively in imaging applications, as they display a greater resistance to photobleaching.

Multiple combinations of bioluminescent donor molecule and the fluorescent acceptor molecule may be used to increase the sensitivity of the detection of tested inhibitor or activator candidates. Criteria which should be considered in determining suitable pairings for BRET is the relative emission/fluorescence spectrum of the acceptor molecule compared to that of the bioluminescent donor molecule. The emission spectrum of the bioluminescent protein should overlap with the absorbance spectrum of the acceptor molecule such that the light energy from the bioluminescent protein luminescence emission is at a wavelength that is able to excite the acceptor molecule and thereby promote acceptor molecule fluorescence when the two molecules are in a proper proximity and orientation with respect to one another. Good spectroscopic properties, e.g., brightness, photostability, may also be considered.

The original BRET system uses the *Renilla* luciferase as donor, EYFP (or Topaz) as the acceptor and coelenterazine derivative as the substrate. These components when combined in a BRET assay generate maximal light in the 475-485 nm range for the bioluminescent protein and the 525-535 nm range for the acceptor molecule, giving a spectral resolution of 40-60 nm. Renilla luciferase generates a broad emission peak overlapping substantially the GFP emission, which in turn contributes to decrease the signal to noise of the system. Various coelenterazine derivatives are known in the art, including coel400a, that generate light at various wavelengths (distinct from that generated by the wild type coelenterazine) as a result of *Renilla* luciferase activity. A skilled person in the art would appreciate that because the light emission peak of the donor has changed, it is necessary to select an acceptor molecule which will absorb light at this wavelength and thereby permit efficient energy transfer. Spectral overlapping between light emission of the donor and the light absorption peak of the acceptor is one condition among others for an efficient energy transfer. Class 3 and 1 GFPs are known to absorb light at 400 nm and re-emit between 505-511 nm. This results in a wavelength difference between donor and acceptor emissions of approximately 111 nm.

Preferred selection of the bioluminescent donor and fluorescent acceptor molecules suitable for use in the intramolecular BRET probe according to the present invention include the combination NanoLuc and mNeonGreen.

As the skilled person in the art will appreciate, the bioluminescent donor molecule and the fluorescent acceptor molecule are inserted such that it makes the ion sensor and/or ion conducting channel subunit active as result in a spatial change to the location and/or dipole orientation of the bioluminescent donor molecule relative to the fluorescent acceptor molecule. The spatial positioning of the ion channel, ion sensor probe, bioluminescent donor molecule and the fluorescent acceptor molecule is such that the resultant fusion channel protein is functional. The energy transfer primarily depends on: (i) an overlap between the emission and excitation spectra of the donor and acceptor molecules, respectively and (ii) the proximity of about 100 Angstroms (A) between the donor and acceptor molecules. The donor molecule in BRET produces light via chemiluminescence, so it is amenable to small animal imaging. In addition, the BRET system does not use an external light excitation source, which provides potentially greater sensitivity in living subjects because of the low signal to noise ratio.

The ion sensor which is used in the probe according to the present invention is a sensor receptive to calcium, potassium, sodium, chloride ions or any sensor receptive to ionic strength. It represents a sensing domain or a sensitive domain which is responsive or sensitive or capable of detecting/sensing presence and levels of a signal ion including for example calcium, potassium, sodium, chloride ions and the like. The sensor or sensing domain then typically changes conformation in response to the binding of a signal ion or molecule or to a changed intracellular condition.

The intramolecular BRET probes according to the present invention are thus useful to monitor ion channel structural conformational changes and ion channel activation in living cells.

According to the present invention, the ion sensor may be chosen among a calcium binding protein, such as the troponin C calcium binding domain or the calmodulin calcium binding protein, a potassium binding protein (KBP), a sodium binding protein, such as the NhAs-1 protein, or a chloride binding protein.

Calcium-sensing proteins or calcium binding proteins are well-known in the art, and they may be any proteins that act in the second messenger system. For example, we can cite Calmodulin, Calnexin, Calreticulin and Gelsolin.

Calmodulin (CaM) (an abbreviation for calcium-modulated protein) is a multifunctional intermediate calcium-binding messenger protein expressed in all eukaryotic cells. It is an intracellular target of the secondary messenger Ca²⁺, and the binding of Ca²⁺ is required for the activation of calmodulin. Once bound to Ca²⁺, calmodulin acts as part of a calcium signal transduction pathway by modifying its interactions with various target proteins such as kinases or phosphatase.

Calnexin (CNX) is a 67kDa integral protein (that appears variously as a 90kDa, 80kDa, or 75kDa band on western blotting depending on the source of the antibody) of the endoplasmic reticulum. It consists of a large (50 kDa) N-terminal calcium-binding lumenal domain, a single transmembrane helix and a short (90 residues) acidic cytoplasmic tail.

Calreticulin also known as calregulin, CRP55, CaBP3, calsequestrin-like protein, and endoplasmic reticulum resident protein 60 (ERp60) is a protein that in humans encoded by the CALR gene. Calreticulin is a multifunctional soluble protein that binds Ca²⁺ ions, rendering it inactive. The Ca²⁺ is bound with low affinity, but high capacity, and can be released on a signal. Calreticulin is located in storage compartments associated with the endoplasmic reticulum (ER) and is considered an ER resident protein.

Gelsolin is an actin-binding protein that is a key regulator of actin filament assembly and disassembly. Gelsolin is one of the most potent members of the actin-severing gelsolin/villin superfamily, as it severs with nearly 100% efficiency.

Potassium sensors or potassium binding proteins are also well-known in the art and it may include any potassium sensor proteins capable of binding positively charged potassium ion and generating a detectable signal upon binding of positively charged potassium ion to the potassium sensor. The binding of K⁺ may for example induce a conformational shift in the potassium sensor and then enable signaling domain to generate a detectable signal. Preferably, the binding of K⁺ to the potassium sensor may induce a conformational shift in the peptide and the signaling domain may then be capable to generate the detectable signal. In a preferred embodiment, the potassium sensor comprises an amino acid sequence of KBP as described by Ashraf et al. (Structure 2016, May 3; 24(5) 741-9). The K⁺ binding protein (KBP) is also known as YgaU, and is characterized by a soluble 16-kDa cytoplasmic protein from *Escherichia coli.* It is a highly specific K⁺ binding protein and is required for normal growth in the presence of high levels of external K. The potassium ion binds exclusively to the BON domain, which upon binding undergoes a conformational change. KBP further comprises the LysM domain which may interact with the BON domain.

Sodium ion binding proteins or sodium sensors may include for example nhaS gene product, NhaS, a protein characterized by binding to and sequestering sodium ion (Na⁺). Also included within the scope are functional fragments of sodium ion binding proteins including NhaS, which fragments are characterized by their ability to bind to sodium ion, see for example US5346815A.

Chloride binding proteins or chloride sensors may include for example the OEC, Chameleon and other chloride requiring enzymes. The polypeptide structure of the OEC has been extensively described *inter alia* by Coleman W.J. (Photosynth Res (1990) 23: 1. https://doi.org/10.1007/BF00030059). The chloride biosensors like YFP-based chloride biosensor Chameleon consists of two fluorescent proteins, CFP and Topaz joined by a flexible polypeptide linker of 24 amino acids as described by Arosio et al. (Front Cell Neurosci. 2014;8:258. 2014 Aug 29. doi:10.3389/fncel.2014.00258) and by Watts et al. (April 10, 2012, https://doi.org/10.1371/journal.pone.0035373). In some cases, the acceptor presents chloride sensitivity and the chloride sensor may thus be, in such cases, the acceptor itself. To this regard, we may cite the YFP-H148Q, a genetically modified YFP photostable fluorescent protein which also presents enhanced chloride sensitivity as described by Zhong S et al. (PLoS One. 2014 Jun 5;9(6):e99095. doi: 10.1371/journal.pone.0099095). Such modified YFP may thus be used as fluorescent acceptor molecule as well as ion chloride sensor for studying activation or inhibition of the ion conducting channels by chloride ion flux.

The present invention thus provides a useful tool to probe for the passage of ions and thus actual activation or inhibition of ion conducting channels as described above. As a result, by multiplexing different BRET-biosensors of such ion conducting channels, the intramolecular novel BRET probes thus offer the possibility to reliably and rapidly test various candidates.

High-throughput screening for ion-channel function requires sensitive, simple assays and instrumentation that will report ion channel activity in living cells. The present invention thus relates to a nucleic acid or polynucleotide comprising a nucleotide sequence encoding a channel fusion subunit comprising a subunit of an ion conducting channel bound to a probe, wherein the probe comprises an ion sensor bound in between to at least a bioluminescent donor molecule and to at least a protein fluorescent acceptor molecule, wherein the ion sensor is configured to undergo a conformational change in the presence of an ion transported by the channel subunit, wherein the probe is bound to the N-terminal, the C-terminal, or within an intracellular or extracellular loop of the channel subunit via either the bioluminescent donor molecule or the protein fluorescent acceptor molecule, and wherein said bioluminescent donor molecule and acceptor molecule are selected so that the emission spectrum of the bioluminescent donor molecule overlaps with the absorbance spectrum of the acceptor molecule, so the light energy delivered by the bioluminescent donor molecule is at a wavelength that is able to excite the acceptor molecule.

The nucleic acid or polynucleotide of the present invention may be inserted into several commercially available expression vectors. Non-limiting examples include prokaryotic plasmid vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for *Pichia pastoris* comprise e.g. the plasmids pAO815, pPIC9K and pPIC3.5K (all Intvitrogen). The nucleic acid or polynucleotide of the present invention referred to above may also be inserted into vectors such that a translational fusion with another polynucleotide is generated. The other polynucleotide may encode a protein which may e.g. increase the solubility and/or facilitate the purification of the fusion protein. Non-limiting examples include pET32, pET41, pET43. The vectors may also contain an additional expressible polynucleotide coding for one or more chaperones to facilitate correct protein folding. Suitable bacterial expression hosts comprise e.g. strains derived from BL21 (such as BL21(DE3), BL21(DE3)PlysS, BL21(DE3)RIL, BL21(DE3)PRARE) or Rosetta®. In preferred embodiments, expression vector according to the invention is a DNA or RNA vector, capable of transforming eukaryotic host cells and effecting stable or transient expression of said TRP channel fusion subunit, and wherein said vector is a plasmid, a virus like adenovirus, adeno associated virus (AVV), lentiviral, Epstein-Barr, Herpes Simplex, Papilloma, Polyoma, Retro, SV40, Vaccinia, any retroviral vector, influenza viral vector and other non-viral vectors including naked DNA, or liposomes.

Transcriptional regulatory elements (parts of an expression cassette) ensuring expression in prokaryotes or eukaryotic cells are well known to those skilled in the art. These elements comprise regulatory sequences ensuring the initiation of the transcription (e.g., translation initiation codon, promoters, enhancers, and/or insulators), internal ribosomal entry sites (IRES) and optionally poly-A signals ensuring termination of transcription and stabilization of the transcript. Additional regulatory elements may include transcriptional as well as translational enhancers, and/or naturally associated or heterologous promoter regions. Preferably, the polynucleotide of the invention is operatively linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Possible examples for regulatory elements ensuring the initiation of transcription comprise the cytomegalovirus (CMV) promoter, SV40-promoter, RSV-promoter (Rous sarcome virus), the lacZ promoter, human elongation factor 1α-promoter, CMV enhancer, CaM-kinase promoter, the *Autographa californica* multiple nuclear polyhedrosis virus (AcMNPV) polyhedral promoter or the SV40-enhancer. For the expression in prokaryotes, a multitude of promoters including, for example, the tac-lac-promoter, the lacUV5 or the trp promoter, has been described. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide.

The present invention is further directed to a recombinant host cell containing an expression vector for expression of the ion conducting channel fusion unit as described above, wherein said vector contains a polynucleotide comprising a nucleic acid comprising a nucleotide sequence encoding a channel fusion subunit comprising a subunit of an ion conducting channel bound to a probe, wherein the probe comprises an ion sensor bound in between to at least a bioluminescent donor molecule and to at least a protein fluorescent acceptor molecule, wherein the ion sensor is configured to undergo a conformational change in the presence of an ion transported by the channel subunit, wherein the probe is bound to the N-terminal, the C-terminal, or within an intracellular or extracellular loop of the channel subunit via either the bioluminescent donor molecule or the protein fluorescent acceptor molecule, and wherein said bioluminescent donor molecule and acceptor molecule are selected so that the emission spectrum of the bioluminescent donor molecule overlaps with the absorbance spectrum of the acceptor molecule, so the light energy delivered by the bioluminescent donor molecule is at a wavelength that is able to excite the acceptor molecule.

Recombinant cells according to the present invention are thus genetically engineered to contain the polynucleotide construct of the ion channel fusion subunit as described above. The cell or host may be any prokaryote or eukaryotic cell and may be preferably a stable cell line. Suitable eukaryotic host may be a mammalian cell, an amphibian cell, a fish cell, an insect cell, a fungal cell or a plant cell. Eukaryotic cell may be an insect cell such as a *Spodoptera frugiperda* cell, a yeast cell such as a *Saccharomyces cerevisiae* or *Pichia pastoris* cell, a fungal cell such as an *Aspergillus* cell or a vertebrate cell. Suitable prokaryotes may be *E. coli* (e.g., *E coli* strains HB101, DH5a, XL1 Blue, Y1090 and JM101), *Salmonella typhimurium, Serratia marcescens, Burkholderia glumae, Pseudomonas putida, Pseudomonas fluorescens, Pseudomonas stutzeri, Streptomyces lividans, Lactococcus lactis, Mycobacterium smegmatis* or *Bacillus subtilis.*

Recombinant host cells according to the present invention thus comprise an expression vector wherein said channel fusion subunit is expressed and is able to co-assemble with other homomeric or heteromeric channel subunits *in vitro* and *in vivo* to form a functional fusion channel. The present invention embodies a process for the production of said channel fusion subunits as described above comprising culturing said recombinant cell according to the invention and expressing said channel fusion subunit.

The present invention also relates to cell-free composition and assays comprising at least ion channel subunit in combination or coupled or bound to an ion sensor nucleotide sequence placed between a nucleotide sequence encoding bioluminescent donor molecule and a nucleotide sequence encoding at least one fluorescent acceptor molecule. Said cell-free composition comprises the ion channel fusion unit or a functional fusion channel as described above, wherein said fusion subunit is embedded in a lipid bilayer, preferably in a bilayer of a liposome. Such lipid bilayer comprises two layers of, typically amphiphilic, with a common hydrophobic bilayer interior and two hydrophilic surfaces. The lipid bilayer can be naturally occurring or artificial. Such lipid bilayer may be a cellular or bio-membrane for example from mammalian or yeast cell membrane, into which voltage-dependent ion channel fusion subunit is inserted.

Methods for preparing cell-free compositions from cells are well-known in the art and consist in obtaining recombinant cells as described above and disrupting the membrane of the cells. These methods generally include repeated cycles of freezing and thawing, grinding, treatment of cells with ultrasound in a sonicator device, homogenization, and use of a French press, the addition of detergent and/or enzymes, glass-bead lysis, differential centrifugation, and several density gradient procedures using a variety of gradient media. These techniques are *inter alia* described in details in "Current Protocols in Protein Science"; John E. Caligan; Ben M. Dunn; Hidde L. Ploegh; David W. Speicher; Paul T. Wingfield; Wiley and Sons). For isolating or preparing cell membrane extracts, a combination of these methods is usually employed. Generally, cells are lysed either by mechanical means, or using detergents, and the membrane fractions isolated via differential centritugation. Liposomes containing recombinant cell as per the invention may be created by disrupting the phospholipid membrane of cells expressing the protein in water, for example by sonication. The phospholipids would reassemble into liposomal spheres containing a core of aqueous solution. Low shear rates would create multilamellar liposomes, which have many layers. Continued high-shear sonication would form smaller unilamellar liposomes, more suited to the application of the present invention.

The present invention further provides a method of assessing whether a test compound functions as a ligand for an ion conducting channel, the method comprising: (i) providing a cell comprising a nucleotide sequence encoding an ion conducting channel subunit bound to a probe, wherein the probe comprises an ion sensor bound in between to at least one bioluminescent donor molecule and at least one fluorescent acceptor molecule, wherein the probe is bound to the N-terminal or the C-terminal, or within an intracellular or extracellular loop of the channel subunit via either the bioluminescent donor molecule or the fluorescent acceptor molecule, and wherein said bioluminescent donor molecule and fluorescent acceptor molecule are selected so that the emission spectrum of the bioluminescent donor molecule overlaps with the absorbance spectrum of the acceptor molecule, making it possible the non-radiative energy transfer between the bioluminescent energy donor and the fluorescent acceptor through nonradiative dipole-dipole coupling; (ii) contacting said cell with a test compound; and (iii) determining the resultant reaction or interaction and output.

The method of screening in real time a compound candidate allow measuring the ion flux and thus the activation or inhibition of ion conducting channel, comprising contacting the candidate with the recombinant host cell or a cell free composition according to the invention, providing a substrate of the bioluminescent donor molecule; (iii) measuring the variation of BRET signal. Such methods allow the screening in real time agonist or antagonist of the ion conducting channel.

In a further aspect, the present invention provides a kit for screening agonist or inhibitor compound candidate of ion conducting channel comprising a nucleic acid or a polynucleotide of the invention, a vector of the invention, a recombinant cell or a host cell of the invention, or a cell-free composition or a composition of the invention, and/or a biosensor of the invention.

Throughout this application, various references are referred to and disclosures of these publications in their entireties are hereby incorporated by reference into this application to more fully describe the state of the art to which this invention pertains.

### EXAMPLES

### Example 1: Construction of an intramolecular probe of 2^{nd} generation

Intramolecular novel BRET probes as showed in Figure 1 have been constructed to measure the ionic flux in the microenvironment of voltage-gated ion channel pore, using ion binding protein sandwiched by YFP and Luc and fused to the intracellular extremities of several ion conducting channel.

### Example 1.1: Construction of potassium binding BRET probe.

The K+-sensitive genetically encoded, bioluminescence resonance energy transfer-(BRET)based probes has been constructed by cloning the cDNA coding the amino acid sequence of KBP, as described by Ashraf et al.(2016) (Structure 2016, May 3; 24(5) 741-9), without the Stop codon, in fusion to the cDNA coding the NanoLuciferase at its 5' extremity and in fusion to the cDNA coding the mNeonGreen fluorescent protein at its 3' extremity, yielding to the NanoLuc-KBP-mNeonGreen BRET probe, or in fusion to the cDNA coding mNeonGreen at its 5' extremity and in fusion to the cDNA coding the NanoLuciferase protein at its 3' extremity, yielding to the NanoLuc-KBP-mNeonGreen BRET probe. A peptide spacer between the N-terminal acceptor or donor protein is not crucial to the operability of the sensor. A RMQDA peptide spacer may thus be optionally added in-between the N-terminal acceptor or donor protein and the KBP protein and a PLAEL peptide spacer is added in-between the KBP protein and the C-terminal donor or acceptor protein.

### Example 1.2: Construction of ionic strenght - sensitive BRET probe

The ionic strenght-sensitive genetically encoded, bioluminescence resonance energy transfer-(BRET) based probes has been constructed by cloning the cDNA coding the ionic strength sensor sequence described by Liu B. et al., (2017) (ACS Chem. Biol. 2017, 12, 2510-2514) and here referenced as SOFI, in fusion to the cDNA coding the NanoLuciferase at its 5' extremity and in fusion to the cDNA coding the mNeonGreen fluorescent protein at its 3' extremity, yielding to the NanoLuc-SOFI-mNeonGreen BRET probe, or in fusion to the cDNA coding mNeonGreen at its 5' extremity and in fusion to the cDNA coding the NanoLuciferase protein at its 3' extremity, yielding to the NanoLuc-SOFI-mNeonGreen BRET probe. A peptide spacer between the N-terminal acceptor or donor protein is not crucial to the operability of the sensor. A RMQDA peptide spacer may thus be optionally added in-between the N-terminal acceptor or donor protein and the calflux protein and a PLAEL peptide spacer is added in-between the calflux protein and the C-terminal donor or acceptor protein.

### Example 1.3: Construction of calcium binding BRET probe

The Ca²⁺-sensitive genetically encoded, bioluminescence resonance energy transfer-(BRET) based probes has been constructed by cloning the cDNA coding the amino acid sequence of calflux, as described by Yang J. et al., (2016) (Nature Communication, 2016, 27;7:13268. doi: 10.1038/ncomms13268), without the Stop codon, in fusion to the cDNA coding the NanoLuciferase at its 5' extremity and in fusion to the cDNA coding the mNeonGreen fluorescent protein at its 3' extremity, yielding to the NanoLuc-calflux-mNeonGreen BRET probe, or in fusion to the cDNA coding mNeonGreen at its 5' extremity and in fusion to the cDNA coding the NanoLuciferase protein at its 3' extremity, yielding to the NanoLuc-calflux-mNeonGreen BRET probe. A peptide spacer between the N-terminal acceptor or donor protein is not crucial to the operability of the sensor. A RMQDA peptide spacer may thus be optionally added in-between the N-terminal acceptor or donor protein and the calflux protein and a PLAEL peptide spacer is added in-between the calflux protein and the C-terminal donor or acceptor protein.

### Example 1.4: Construction of chloride binding BRET probe

The chloride-sensitive genetically encoded, bioluminescence resonance energy transfer-(BRET) based probes has been constructed by cloning the cDNA coding the amino acid sequence of YFP or YFP variant in fusion to the cDNA coding the NanoLuciferase at its 3' extremity and by introducing point mutations conferring chloride sensitivity to YFP construct (for instance mutations H148Q and I152L, as described by Galietta et al., FEBS letters, 2001, Jun 22;499(3):220-4. DOI:10.1016/s0014-5793(01)02561-3).

### Example 2: Expression vectors carrying the sensor-based BRET probes

The expression of this protein in target mammals' cells is made possible by the transfection of a vector of expression comprising the cDNA encoding a fusion protein comprising the channel of interest and the ion sensor using the BRET technology.

The transitory or stable expression of the vector in targeted mammalian cells allowed the direct measuring of the activity of the targeted channel by the BRET probe according to the invention. This protein allowed the emission of signal (light) during the addition of the luciferase substrate, such as Cœlenterazine H. Luciferase catalyzed the oxidation of the substrate. This biochemical reaction generates a light emission at a wavelength which is characteristic of the couple luciferase / substrate. If the acceptor which has an excitation spectrum compatible to the emission spectrum of the couple luciferase / substrate, is in proximity with the proper orientation, a non-radiative transfer of energy (BRET signal) will occur between the donor and the acceptor. This transfer of energy will be characterized by the fluorescence of the acceptor at its wavelength of emission (Figure 2). Typically, in order to have a transfer of energy between a donor and compatible acceptor, the 2 groups must be at a distance of around 10nm from each other's, which is a distance compatible for the interaction's proteins-proteins.

### Example 3: calcium sensitive BRET probe targeting the TRPV1 channel

The intramolecular BRET probe : hTRPV1-nLuc-Calflux-YFP has been prepared wherein the DNA sequence encoding the human protein TRPV1 is fused in C-terminal to a calcium sensor, said sensor being sandwiched between the nano luciferase and YFP or mNeonGreen (Calflux probe, Yang J. et al., Nature Communication, 2016, 27;7:13268. doi: 10.1038/ncomms13268.). Applicant showed that the basal BRET signal of the probe hTRPV1-nLuc-Calflux-YFP (or mNeonGreen) is modulated in dose-dependent manner further to the activation of the cells by Capsaïcin (CAPS), which is the principal agonist of TRPV1 (Fig. 4A).

This activation was absolutely specific of the passage of the ions across TRPV1 for the following reasons:
- AMG517, which is a TRPV1 antagonist, shifted the dose-response to CAPS to the right indicating a competitive inhibition as expected (Fig. 4A);
- The nLuc-Calflux-YFP probe when expressed alone without being fused to TRPV1 did not respond to the stimulation by CAPS in absence of co-expressed TRPV1 ion channel (Fig. 4A);
- The hTRPV1-nLuc-Calflux D13A/D51A-YFP probe containing a double-mutation making the Calflux probe insensitive to calcium, did not respond to CAPS stimulation. This control was important to show that conformational change induced by CAPS on the TRPV1 channel did not participate to the increase of the observed BRET signal (Fig. 4A);
- The non-specific diffusion of calcium into cells by using ionomycin (a calcium ionophore), did not cause a dose-dependent increase of the BRET signal, as measured with the free nLuc-Calflux-mNeonGreen probe, but only caused a marginal increase of the signal obtained with the TRPV-nLuc-Calflux-mNeonGreen probe (Fig. 4B).

These data clearly evidenced that the herein described BRET probe targeting the TRPV1 channel specifically reflected the passage of ions across the TRPV1 channel and thus reflected TRPV1 real activity.

Analysis of this dose-response curve showed that CAPS induced TRPV1 activation with an EC50 of 328±33 nM, indicating a sensitivity of the BRET probe to CAPS with pharmacological properties similar to those found in the scientific literature using patch-clamp.

### Example 4: Sensor-based BRET Probe targeting canal KCa_{2.3}

The novel BRET probe has been constructed to measure the activity of potassium channel SK3 (KCa_{2.3}) by coupling the cDNA of the SK3 channel to a potassium sensor (protein KBP, Ashrafet KU al., 2016, Structure 24, 741-749) sandwiched in between luciferase and mNeonGreen (SK3-nLuc-KBP-mNeonGreen, See Fig. 5A).

The SK channels are a family of potassium channel activated by the increase of the cytoplasmic calcium concentration. Opening of the SK3 channels induces a small outward flux of potassium ions diffusing through the opened pore according to the potassium ion electrochemical gradient. Following addition of ionomycin at different concentrations, in order to induce a cytosolic increase of calcium and subsequently activating the SK3 channel, a decrease of the BRET signal measured in HEK293T cells expressing the SK3-nLuc-KBP-mNeonGreen probe indicating an efflux of potassium outside the cells (Fig. 5B). The BRET sensor is based on KBP which has been published (Bischof H et al, 2017, Nat Commun. 8(1):1422), but this sensor was never coupled to a channel in order to specifically measure the activity of the channel as presented in this invention.

### Example 5: Sensor-based BRET Probe targeting receptor P2X2

We have constructed a rP2X2-nLuc-Calflux-YFP probe wherein the DNA sequence encoding the rat protein P2X2 is fused in C-terminal to a calcium sensor sandwiched in between the nanoluciferase and YFP or mNeonGreen (Calflux probe, Yang J. et al., Nature Communication, 2016, 27;7:13268. doi: 10.1038/ncomms13268.).

We were able to show that the basal BRET signal of the rP2X2-nLuc-Calflux-YFP probe (or mNeonGreen) was modulated in a dose-dependent manner following the activation of the cells by ATP, which is the natural agonist of P2X2 (Fig. 6A). This activation is specific of the passage of the ions through P2X2 for the following reasons:
1/ when the nLuc-Calflux-YFP probe is expressed alone without being fused to P2X2, the BRET probe does not respond to the ATP stimulations (Fig. 6B);
2/ when the nLuc-Calflux-YFP probe is fused to the TRPV1 channel, this probe does not respond to the ATP stimulations (Fig. 6C) but respond to the capsaicine (CAPS) stimulation, which is the agonist of TRPV1.

Analysis of the dose-response curve indicated that ATP activated the P2X2-NLuc-CalFlux-YFP probe with an EC50 of 888±274 nM, indicating that the ATP potency to activate P2X2 BRET probe is similar to the ATP potency to activate native P2X2 as found in the scientific literature using conventional techniques.

### Example 6: Sensor-based BRET Probe targeting NMDA receptors

We have constructed a novel BRET probe allowing to measure the activity of NMDA receptors by coupling the cDNA of the NR1 subunit to the BRET sensor nLuc-Calflux-YFP (Fig. 7). NMDA receptors are heterotetramers comprising 2 NR1 subunits and 2 NR2 or NR3 subunits. The subunits NR1, NR2 or NR3 expressed separately cannot form functional receptors.

We were able to show that the BRET signal generated when expressing the BRET probe NR1-nLuc-Calflux-YFP alone is stable and is not modulated by increasing dose of NMDA (Fig. 7A). However, the coexpression of this probe with the subunits NR2A (Fig. 7B) or NR2B (Fig. 7C) generated functional receptors and an increase of the signal was observed in HEK293T cells co-expressing NR1-nLuc-Calflux-YFP and NR2A subunits following cells' stimulation with NMDA. Analysis of the dose-response curve indicated that NMDA activated the NR1-nLuc-Calflux-YFP / NR2A and NR1-nLuc-Calflux-YFP / NR2B ion channels with EC50 of 79.7 µM +/- 17.2 µM and 12.3 +/- 7.32 µM respectively. These data were perfectly in line with the scientific literature thus showing that heteromeric receptors comprising NR2A subunits are less sensitive to NMDA than receptors comprising NR2B subunits.

### Example 7: Sensor-based BRET Probe targeting calcium-permeable cation channel TRPM8

The intramolecular BRET probe: hTRPM8-nLuc-Calflux-YFP has been prepared wherein the DNA sequence encoding the human protein TRPM8 is fused in C-terminal to a calflux-based calcium BRET sensor, said sensor being sandwiched between the nano luciferase and YFP or mNeonGreen.

### Example 8: Sensor-based BRET Probe targeting calcium-permeable cation channel TRPV4

The intramolecular BRET probe: hTRPV4-nLuc-Calflux-YFP is prepared wherein the DNA sequence encoding the human protein TRPV4 is fused in C-terminal to a calflux-based calcium BRET sensor.

### Example 9: Sensor-based BRET Probe targeting sodium channel Nav1.7

The intramolecular BRET probe: Nav1.7-nLuc-SOFI-YFP is prepared wherein the DNA sequence encoding the human protein TRPV4 is fused in C-terminal to a SOFI-based ionic strength BRET sensor.

### Example 10: Sensor-based BRET Probe targeting potassium channel Kv7.1

The intramolecular BRET probe: Kv7.1-nLuc-KBP-YFP is prepared wherein the DNA sequence encoding the human protein TRPV4 is fused in C-terminal to a KBP-based potassium BRET sensor.

### Example 11: Sensor-based BRET Probe targeting one chloride channel LRRC8A

The intramolecular BRET probe: LRRC8A-nLuc-YFPH148Q;I152L is prepared wherein the DNA sequence encoding the human protein LRRC8A is fused in C-terminal to a Chloride-based BRET sensor comprising NanoLuc and a chloride sensitive YFP mutant (YFP H148Q;I152L).

## Claims

1. A channel fusion subunit comprising an ion conducting channel subunit bound to a probe, wherein the probe comprises an ion sensor bound in between to at least one bioluminescent donor molecule and at least one fluorescent acceptor molecule, wherein the probe is bound to the N-terminal, C-terminal, or within an intracellular or extracellular loop of said channel subunit via either the bioluminescent donor molecule or the fluorescent acceptor molecule, and wherein said bioluminescent donor molecule and fluorescent acceptor molecule are selected so that the emission spectrum of the bioluminescent donor molecule overlaps with the absorbance spectrum of the acceptor molecule, making it possible the non-radiative energy transfer between the bioluminescent energy donor and the fluorescent acceptor through nonradiative dipole-dipole coupling.

2. The channel fusion subunit of claim 1, wherein the ion conducting channel is a voltage-dependent ion channel or a ligand-dependent ion channel.

3. The channel fusion subunit of any one of the preceding claims, wherein the ion sensor is a sensor to calcium, potassium, sodium, chloride ions or a sensor to ionic strength.

4. The channel fusion subunit of any one of the preceding claims, wherein the ion sensor is chosen among a calcium binding protein, such as the troponin C calcium sensitive domain or the calmodulin calcium binding protein, a potassium binding protein (KBP), a sodium binding protein, such as the NhAs-1 protein, or a chloride binding protein.

5. The channel fusion subunit of any one of the preceding claims, further comprising a linker between the ion conducting channel subunit and the probe.

6. The channel fusion subunit of any one of the preceding claims, wherein the bioluminescent donor molecule is a luciferase chosen among Renilla luciferase, Firefly luciferase, Coelenterate luciferase, North American glow worm luciferase, click beetle luciferase, a railroad worm luciferase, Gaussia luciferase, Aequorin, Arachnocampa luciferase, Nanoluciferase derived from deep sea shrimp *Oplophorus gracilirostris,* or a biologically active variant or fragment thereof, GLuc, NanoLuc (NLuc), MLuc7, HtLuc, LoLuc, PaLuc1, PaLuc2, MpLucl, McLucl, MaLuc1, MoLuc1, MoLuc2, MLuc39, PsLucl, LocLucl-3, HtLuc2 Renilla, TurboLucl6 (TLuc) or homologs or orthologs thereof or mutants or functional derivatives thereof.

7. The channel fusion subunit of any one of the preceding claims, wherein the bioluminescent donor molecule is a non-luciferase bioluminescent protein chosen among β-galactosidase, lactamase, horseradish peroxidase, alkaline phosphatase, β-glucuronidase, or β-glucosidase.

8. The channel fusion subunit of any one of the preceding claims, wherein the acceptor molecule is a protein chosen among green fluorescent protein (GFP), variant of green fluorescent protein (GFP10), blue fluorescent protein (BFP), cyan fluorescent protein (CFP), yellow fluorescent protein (YFP), enhanced GFP (EGFP), enhanced CFP (ECFP), enhanced YFP (EYFP), GFPS65T, mAmetrine, LSS-mOrange, LSS-mKate, Emerald, Topaz, GFPuv, destabilised EGFP (dEGFP), destabilised ECFP (dECFP), destabilised EYFP (dEYFP), HcRed, t-HcRed, DsRed, DsRed2, mRFPI, pocilloporin, Renilla GFP, Monster GFP, paGFP, Kaede protein, mNeonGreen derived from Branchiostoma lanceolatum or a Phycobiliprotein, or a biologically active variant or fragment of any one thereof, TagCFP, mTagCFP2, Czurite, ECFP2, mKalamal, Sirius, Sapphire, T-Sapphire, ECFP, Cerulean, SCFP3C, mTurquoise, mTurquoise2, monomeric Midoriishi-Cyan, TagCFP, mTFPI, EGFP, Emerald, Superfolder GFP, Monomeric Czami Green, TagGFP2, mUKG, mWasabi, Clover, Citrine, Venus, SYFP2, TagYFP, Monomeric Kusabira-Orange, 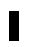 ηKOₖ, mK02, mOrange, mOrange2, mRaspberry, mCherry, mStrawberry, mScarlet, mTangerine, tdTomato, TagRFP, TagRFP-T, mCpple, mRuby, mRuby2, mPlum, HcRed-Tandem, mKate2, mNeptune, NirFP, TagRFP657, IFP1.4 and iRFP or a biologically active variant or fragment of any one thereof.

9. A recombinant cell expressing the channel fusion subunit of any one of the preceding claims.

10. A method for screening a candidate compound capable of modulating ion flux through a channel, comprising:
contacting the candidate compound with the recombinant cell of claim 9;
providing a substrate of the bioluminescent donor molecule; and
measuring a variation of bioluminescent resonance energy transfer.

11. A nucleic acid comprising a nucleotide sequence encoding a channel fusion subunit comprising a subunit of an ion conducting channel bound to a probe, wherein the probe comprises an ion sensor bound in between to at least a bioluminescent donor molecule and to at least a protein fluorescent acceptor molecule, wherein the ion sensor is configured to undergo a conformational change in the presence of an ion transported by the channel subunit, wherein the probe is bound to the N-terminal, the C-terminal, or within an intracellular or extracellular loop of the channel subunit via either the bioluminescent donor molecule or the protein fluorescent acceptor molecule, and wherein said bioluminescent donor molecule and acceptor molecule are selected so that the emission spectrum of the bioluminescent donor molecule overlaps with the absorbance spectrum of the acceptor molecule, so the light energy delivered by the bioluminescent donor molecule is at a wavelength that is able to excite the acceptor molecule.

12. The nucleic acid of claim 11, wherein the ion conducting channel is a voltage-dependent ion channel or a ligand-dependent ion channel, wherein the ion sensor is a sensor to calcium, potassium, sodium, chloride ions or a sensor to ionic strength, optionally comprising a linker sequence between the nucleotide sequence encoding the ion conducting channel subunit and the nucleotide sequence encoding the probe.

13. The nucleic acid of any one of claims 11 to 12, wherein the ion sensor is chosen among the troponin C calcium binding domain or the calmodulin calcium binding protein, a potassium binding protein (KBP), a sodium binding protein, such as the NhAs-1 protein, or a chloride binding protein.

14. The nucleic acid of any one of claims 11 to 13, wherein the bioluminescent donor molecule is a luciferase chosen among Renilla luciferase, Firefly luciferase, Coelenterate luciferase, North American glow worm luciferase, click beetle luciferase, a railroad worm luciferase, Gaussia luciferase, Aequorin, Arachnocampa luciferase, Nanoluciferase derived from deep sea shrimp *Oplophorus gracilirostris,* or a biologically active variant or fragment thereof, GLuc, NanoLuc (NLuc), MLuc7, HtLuc, LoLuc, PaLuc1, PaLuc2, MpLucl, McLucl, MaLuc1, MoLuc1, MoLuc2, MLuc39, PsLucl, LocLucl-3, HtLuc2 Renilla, TurboLucl6 (TLuc) or homologs or orthologs thereof or mutants or functional derivatives thereof, or wherein the bioluminescent donor molecule is a non-luciferase bioluminescent protein chosen among β-galactosidase, lactamase, horseradish peroxidase, alkaline phosphatase, β-glucuronidase, or β-glucosidase.

15. The nucleic acid of any one of claims 11 to 14, wherein the acceptor molecule is a protein chosen among green fluorescent protein (GFP), variant of green fluorescent protein (GFP10), blue fluorescent protein (BFP), cyan fluorescent protein (CFP), yellow fluorescent protein (YFP), enhanced GFP (EGFP), enhanced CFP (ECFP), enhanced YFP (EYFP), GFPS65T, mAmetrine, LSS-mOrange, LSS-mKate, Emerald, Topaz, GFPuv, destabilised EGFP (dEGFP), destabilised ECFP (dECFP), destabilised EYFP (dEYFP), HcRed, t-HcRed, DsRed, DsRed2, mRFPI, pocilloporin, Renilla GFP, Monster GFP, paGFP, Kaede protein, mNeonGreen derived from Branchiostoma lanceolatum or a Phycobiliprotein, or a biologically active variant or fragment of any one thereof, TagCFP, mTagCFP2, Czurite, ECFP2, mKalamal, Sirius, Sapphire, T-Sapphire, ECFP, Cerulean, SCFP3C, mTurquoise, mTurquoise2, monomeric Midoriishi-Cyan, TagCFP, mTFPI, EGFP, Emerald, Superfolder GFP, Monomeric Czami Green, TagGFP2, mUKG, mWasabi, Clover, Citrine, Venus, SYFP2, TagYFP, Monomeric Kusabira-Orange, 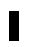 ηKOk, mK02, mOrange, mOrange2, mRaspberry, mCherry, mStrawberry, mScarlet, mTangerine, tdTomato, TagRFP, TagRFP-T, mCpple, mRuby, mRuby2, mPlum, HcRed-Tandem, mKate2, mNeptune, NirFP, TagRFP657, IFP1.4 and iRFP or a biologically active variant or fragment of any one thereof.
